(19) Europäisches Patentamt
European Patent Office
Office européen des brevets

(11) **EP 4 190 371 A1**

(12) **EUROPEAN PATENT APPLICATION**

(43) Date of publication:
**07.06.2023 Bulletin 2023/23**

(21) Application number: **22210825.0**

(22) Date of filing: **01.12.2022**

(51) International Patent Classification (IPC):
**A61M 1/00** *(2006.01)* **A61F 5/44** *(2006.01)*
**A61M 25/00** *(2006.01)*

(52) Cooperative Patent Classification (CPC):
**A61M 1/70; A61M 25/0017;** A61M 2210/1085

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **03.12.2021 JP 2021197398
03.06.2022 JP 2022090695**

(71) Applicant: **Fukui, Kazuhiko
Nasushiobara-shi, Tochigi (JP)**

(72) Inventor: **Fukui, Kazuhiko
Nasushiobara-shi, Tochigi (JP)**

(74) Representative: **Berggren Oy
P.O. Box 16
Eteläinen Rautatiekatu 10A
00101 Helsinki (FI)**

(54) **SUSTAINED DRAIN SYSTEM CIRCUIT AND QUALITY CONTROL SYSTEM THEREFOR**

(57) A sustained drain system circuit and a sustained drain quality control system are constructed in which no mechanical or electrical driving and control mechanism is required at all, siphoning automatically occurs when some volume of urine is retained in the urinary bladder by the mass of urine, its potential energy, intrinsic pressure of filling and contraction of the urinary bladder, and abdominal pressure, and sustained drain continues even with a portion with a height difference higher than a drain source. In the sustained drain system circuit, a fluid is caused to be sustainedly drained by siphoning from an intracorporeal elastic closed space that can extend and contract as a drain source. The circuit is a siphoning circuit in which an inner diameter of a pipe space is designed so that a siphoning volume condition is satisfied and the fluid is drained as filling without a gap also when being drained inside the pipe space, siphoning occurs once a pressure difference P exceeds a Ps value balanced with an initial siphoning resistance Rs. Even if a second height difference H2 occurring a loop after a first height difference HI is present, drain continues without creating negative pressure by elasticity of the drain source and complete draining is made.

Fig. 6

**Description**

BACKGROUND OF THE INVENTION

Field of the Invention

The present invention is controlled by a control system which clarifies a technique and its principles, the technique of causing a fluid to be drained by siphoning from an intracorporeal elastic closed space as a drain source via a portion higher than the drain source to an extracorporeal space as a drain destination by getting over two or more height differences, establishes and checks specifications of the technique, and assures quality. The present invention involves processes to be implemented in other medical devices and other products, and is versatile.

Quality can be assured and controlled by involving a test method and a system. In the test method, it is determined whether a sustained drain system circuit clears specifications, the sustained drain system circuit being designed and manufactured by setting use conditions by a clinical loop model so that the fluid passes once through a position higher than the drain source in an assumed actual clinical use scene and establishing specifications accordingly. In the system, specifications of an artificial urinary bladder and an artificial fluid for checking and determining a product use limit due to various clinical variable factors are further determined and, eventually, the quality of the product is checked and assured. This method and system are inevitably required for danger-prone medical device products. For any medical device, the quality control system based on scientific systems thinking is required to seriously study and inspect whether use conditions in clinical practice are truly satisfied, each quality condition is satisfied, and quality is assured.

Description of the Related Art

In medical practice, it is required to cause a fluid to be drained from an intracorporeal closed space as a drain source to an extracorporeal space with a height difference higher than the drain source as a drain destination. So far, effects provided by the height difference with actual user conditions have not been considered at all.

For example, a drain tube urine collection bag circuit (hereinafter abbreviated as a urine collection circuit) in which urine retained in the urinary bladder is drained (discharged) to the outside of the body by using an indwelling bladder balloon catheter (Foley) (hereinafter referred to as "drain guide material"), which is connected to a urine collection bag by a urine collection tube (hereinafter referred to as a "drain tube") is indicated as "precision-urinometer-equipped closed urine collection bag type 12 Fr to 18 Fr" and has been widely used. Fr is a unit indicating an outer diameter, and 1 Fr =1/3 mm. Thus, the outer diameters of the drain guide material and the indwelling bladder catheter from 4 mm to 6 mm and description about the outer diameter (thickness) are provided, but the inner diameters, which are to be considered in the sustained drain system circuit of the present invention, are not described at all. Moreover, although incapable of sustained drain or capable of drain but only intermittently, the urine collection circuit does not have description about a minimum necessary volume and is labeled as "precision-urinometer-equipped". Furthermore, it is labeled as "closed", which increase peripheral resistance. Still more, description about the inner diameter of the drain tube is not provided at all.

In the above, use conditions in actual clinical and caring practice are not considered at all. In practice, a portion with a height difference is present midway between the drain source and the drain destination, the portion being higher than the drain source, and the fluid does not flow directly from the drain source to the drain destination.

The circuit is of no use unless the fluid is drained as getting over the height difference with the portion being higher than the drain source. This fact and dangerousness are not described at all in the operation manual of the conventional product.

Also in medical and caring practice, the conventional circuit has been continuously used without awareness of detrimental effects and dangerousness.

Thus, functions intrinsically expected are not exerted. Also, appropriate use is not practiced by using patients and nurses in charge of control and nursing and in caring practice or the like, and the purpose of letting urine at each time drained and appropriately measuring its urine volume is not achieved.

In the Japanese Industrial Standard (JIS) T3214: 2011 for urethral catheters, various specifications and use notices of balloon catheters as drain guide materials are described, but items about urine collection (urine guide, inlet) tubes and urine collection bags are not described, and specifications and information about their functions and structures are not described at all.

In JIS T3215: 2011, the scope of application of "drainage catheters and accessory devices" includes drainage catheters and accessory devices designed to discharge a fluid or gas to the outside of the body by gravity or negative pressure, but does not include urinary tract catheters. The group of urinary tract catheters is excluded, and specifications for checking full drain are not described.

The following are the JIS for medical devices (as of June 8, 2006).

T3214 Urethral catheters
T3215 Drainage catheters and accessory devices
T3216 Tubes and catheters for nephrostomy and cystostomy
T3247 Catheters and introducer kits designed for ureter, and urethral dilatation balloon catheters

The specifications of urine collection circuits are not described in any of the above.

Also, the indwelling bladder catheter as a medical device is regulated by "the Act on Securing Quality, Efficacy and Safety of Products Including Pharmaceuticals and Medical Devices". Its marketer obtains an approval as a medical device marketer, makes a medical device marketing registration, and obtains approval or certification for medical device marketing for each medical device to be handled, and then launches the product to the market.

In the following, medical device approval or certification numbers of relevant products commercialized by thirteen companies authorized as medical device marketers in Japan are listed. In the operation manuals and notices of all of the products, notices about the indwelling bladder balloon catheter are written, but the specifications and notices about the general urine collection circuit are not written at all.

16100BZZ01536000 223ACBZX00083000
226AIBZX00064000
229AGBZX00035000 JAN4562246172492 301AIBZX00011000
301AIBZX00010000
218AIBZX00059000
15800BZZ01130000
20900BZY01023000 20900BZY01024000 21200BZY00110000
20700BZZ01034000
20900BZY00070000
21100BZY00388000
221ADBZX00071000
20200BZZ00775
220AIBZX00057000

According to the experience of the inventor, the conventional drain tube urine collection bag circuit (hereinafter referred to as a urine collection circuit) connected to a catheter indwelled in the urinary bladder is of a fully-sealed type (closed type), and urine does not sustainedly flow out at all if the circuit is left as it is. This cannot achieve appropriate urine volume measurement also in an important postoperative period.

Midway through the circuit, clogging may occur to inhibit drain from the urine bladder and cause urine to be retained in the urinary bladder, thereby giving the user painful feelings of pressure on the urinary bladder never experienced ever before.

Moreover, urine clogging occurs midway through the circuit to cause an airlocked state and an artificial anuria state, thereby increasingly damaging and injuring the function of the urinary bladder. This may cause not only neurogenic bladder but also a danger of hydronephrosis by affecting the ureter and the kidney retrogradely by the chronic use of the ailing urinary bladder for a long period of time.

To drain urine, potential energy is provided to urine clogging as a physical condition and a vibration stimulus is further provided to the tube to perform operation of promoting urine drain.

Furthermore, even if urine flows, urine clogging may be formed midway again unless it is confirmed that urine surely drips down to the final urine collection bag.

For this reason, the user in an ICU has to repeat urine drain operation some times to release uncomfortable feelings in the urinary bladder and then sleep, wake up with feelings of pressure on the urinary bladder, perform pressure releasing operation, and then sleep.

BRIEF SUMMARY OF THE INVENTION

To address these current circumstances, the inventor of the present invention has constructed a sustained drain system circuit and a sustained drain quality control system as follows. In these circuit and system, no special auxiliary equipment is required except an open-ended drip chamber, no mechanical or electrical driving and control mechanism is required, retained urine can be sustainedly drained under the law of universal gravitation by the mass of urine, its potential energy, intrinsic pressure of filling and contraction of the urinary bladder, and abdominal pressure even if a portion midway through the circuit is higher than the drain source to some extent. In practical clinical sites, the natural urinating function of the urinary bladder can be maintained. When some volume of urine is retained in the urinary bladder, siphoning automatically occurs, urine is sustainedly drained even with some height difference midway, and complete draining is achieved. That is, sustained complete drain is made in a forward direction without anuresis, residual urine, and excessive loads on the urinary bladder, even with a height difference between the drain source and the drain destination. As a result, the risk of infection is reduced, disuse of the function of the urinary bladder and renal dysfunction are prevented, and recovery of the function of the urinary bladder and the bodily function is pursued.

That is, to create a quality-assured system circuit in which urine is sustainedly and continuously drained in a forward direction without backflow even under use conditions in actual practice and urine is completely drained without being stopped or retained in the urinary bladder or portions midway through the circuit, the inventor has constructed a method of checking specifications and quality of the circuit and, furthermore, a system which checks and controls the circuit whether it can be driven in various properties of the fluid and functions of the urinary bladder.

A siphoning sustained drain system circuit by a clinical loop model and its conformance check test are described.

First aspect of the present invention:
Regarding the structure of the sustained drain system circuit and necessary conditions [0024]

Second aspect of the present invention:
Regarding clinical loop model conditions [0035]

Third aspect of the present invention:
Regarding initial siphoning pressure [0040]

Fourth aspect of the present invention:
Regarding height difference resistance due to the use environment and resistance due to the structure of the circuit [0043]

Fifth aspect of the present invention:
Regarding the minimum volume and initial siphoning pressure [0044]

Sixth aspect of the present invention:
Regarding initial siphoning pressure and sustained drain (siphoning) [0045]

Seventh aspect of the present invention:
Regarding an initial siphoning pressure measurement method and a simple complete drain check test [0052]

Eighth aspect of the present invention:
Regarding variability of the specifications and utilization for rehabilitation [0058]

Ninth aspect of the present invention:
Regarding a reference artificial urinary bladder, and a reference artificial urine and a reference concentrated urine sugar [0062]

Tenth aspect of the present invention:
Regarding a loading test [0066]

To solve the problems described above, the first aspect of the present invention is directed to the structure of the sustained drain system circuit and necessary conditions.

The first aspect of the invention is directed to a sustained drain system (SDS) in which a fluid is caused to be sustainedly drained by the siphoning theory from an intracorporeal elastic closed space as a drain source to an extracorporeal space as a drain destination even with a portion with a first height difference higher than the drain source and several second height differences thereafter. The system includes a drain guide material provided to cause the fluid to be drained from the drain source, a drain tube for moving the fluid from the drain guide material to the drain destination, and a structure formed of an open-ended drip chamber and a closed retention bag. The first aspect presents a method of clarifying design and driving conditions of a system circuit to exert functions of siphoning, sustained drain, and complete draining even under clinical loop model conditions with the first and second height differences obtained by modeling actual use conditions, thereby improving the conventional product. Also, a sustained drain control system is used to check whether the circuit conforms to defined specifications, assure quality of the circuit, and clarifying a use limit and points to be attended to, thereby creating a sustained drain system circuit with security, safety, and usefulness.

That is, in the sustained drain system circuit, a fluid is caused to be sustainedly drained from an intracorporeal elastic closed space that can extend and contract as a drain source. The circuit includes a drain guide material provided to cause the fluid to be drained from the drain source, a drain tube for moving the fluid from the drain guide material to a drain destination, and a closed retention bag including an open-ended drip chamber. When a minimum volume to produce an initial siphoning pressure Ps as an initial pressure difference for siphoning to get over a height difference resistance Rh of a first height difference (loop) Hl in a clinical loop model condition in consideration of an actual clinical use environment condition to start sustained drain and to fill a necessary portion of the elastic closed space as the drain source is Vmin and a volume filling a tube space of a height difference of a siphoning boundary where the fluid gets over a peak of the first height difference 1 (loop 1) by that pressure Ps to start drain is Vloop, a relation in a portion midway through the circuit between an inner space amount of the drain guide material Vhg and an inner space amount of the drain tube is Vht is represented as follows.

[Equation 12]

$$V\,min \geqq Vloop = V\,hg + V\,ht$$

The invention according to the first aspect is directed to a siphoning circuit which satisfies the above siphoning volume conditional expression and in which the inner diameter of the tube space is designed so that the fluid is drained as filling (siphoning) without a gap while draining inside the pipeline. This sustained drain system circuit has a function in which siphoning occurs once the pressure difference P exceeds Ps balanced with the initial siphoning resistance Rs, and drain continues by the siphoning theory without creating negative pressure by elasticity of the drain source even if a second height difference is further present midway, allowing complete draining.

However, even if

[Equation 13]

$$P-R\,s > 0$$

is satisfied, in a non-siphoning circuit in which a fluid does not fill the circuit to cause a gap and drips, clogging is formed even with a slight second height difference, causing an airlocked state and disabling sustained drain. For this reason, it is required to design, in advance, the diameter and length of the drain tube in which siphoning occurs without causing a gap in accordance with the specific gravity (density) and consistency (viscosity) of the fluid when the drain circuit starts initial siphoning under the clinical loop model conditions, and establish specifications of a siphoning check test for actually performing siphoning and checking complete draining. A circuit which has cleared the specification test is quality-assured as a sustained drain system circuit, which is assured by the sustained drain control system which also controls its limitation and further improvement in quality.

That is, the sustained drain system circuit is quality-controlled, having its function and quality assured by the drain quality control system also under use conditions and environment in a practical clinical situation and in caring practice.

There are four necessary conditions for driving this sustained drain system circuit with its structure and functions defined as described above, and the sustained drain system circuit can fully function only after these four conditions are all satisfied.

These necessary conditions for SDS (NCSDS) are as follows.

First condition: The circuit terminal of a drain destination is open.

That is, the peripheral resistance of the retention bag is "Equation 10 (Rp=0)".

Second condition: The drain tube has a tube diameter which allows its inside to be filled with the fluid without a gap, and is required to be thinner than that of the conventional circuit.

Third condition: A total of a volume Vg of the drain guide material and a volume Vt of the drain tube is smaller than the siphoning minimum volume Vmin. That is, the circuit can be sufficiently filled with the siphoning minimum volume.

$$"(\text{Equation 1 } (Vmin=Vo+Vhg+Vht \geq Vg+Vt>Vloop=Vhg+Vht)"$$

That is, a volume for producing an initial siphoning pressure in a urinary bladder state under bodily and mental conditions is Vmin (definition of siphoning), the conditions being formed of the initial siphoning pressure Ps which gets over the first loop height difference, the minimum siphoning volume Vmin, an internal pressure P of the elastic closed space, and a volume Vo of the drain source at the instant of siphoning and the volume Vhg occupying the drain guide material and the volume Vht occupying the drain tube at that time.

Fourth condition: Siphoning occurs when the drain driving pressure P is larger than the initial siphoning pressure Ps balanced with the siphoning pressure Rs (first height resistance) at the siphoning boundary to start drain, and drain continues.

$$"(\text{Equation 11 } (Ps \geq Rs=Rh+Rg+f(Lht, rt)>Rg+Rt)"$$

In this relational expression, the height difference resistance Rh is a resistance occurring by the use environment (first height difference), and is not caused by a factor derived from the circuit itself. Elements to be contrived in the circuit itself are limited to three: the length of the pipeline, the radius of the inner diameter, and the coefficient of friction. The indwelling bladder catheter conventionally being used as a drain guide material is almost-perfect medical equipment, and the inner diameter and the length of the pipeline do not have much allowance to be changed. Reducing the pipeline resistance as much as possible is the only way we can do. For this, the only way is decreasing the coefficient of friction of the material or the surface of the inner space as much as possible. On the other hand, by further contriving a urine collection tube as a drain tube in view of its material to reduce its inner diameter, length, and the coefficient of friction in consideration of the use conditions and environment, the initial siphoning resistance can be reduced, and there is a possibility of reducing the load on the urinary bladder and the body. In other words, this SDS circuit depends on how this drain tube is contrived so as to have small resistance and be light in weight and usable with ease in a clinical situation. There is a possibility that patents and know-hows regarding artificial vessels may be useful. At least low-quality tubes clogged with bloody urine do not have to be provided. Ideally, a circuit is desired like an artificial vessel almost without friction resistance and not clogged with viscous concentrated urine, urine sugar, urine protein, bloody urine, or the like even the circuit is thin to allow sustained drain.

These four conditions are essential conditions for constructing the sustained drain system. When these conditions are satisfied, the siphoning volume Vmin is retained to automatically get over the height difference by contractive force and abdominal pressure of the urinary bladder itself to cause sustained drain to be continued, and the fluid is fully drained from the drain source. The fluid does not remain in the drain source and a portion midway through the drain circuit, and is completely drained and discharged. That is, by using the urine collection circuit and the open-ended drip chamber initially designed "Equation 10 (Rp=0)", the conventional drain guide material can be used as it is. And, the three elements for improvement of the drain tube are important, that is, the length of the tube, the radius of the tube, and the coefficient of friction of the tube. By using these three elements, Vmin is determined, and the initial siphoning pressure is also defined.

The increasing fluid in the drain source sustainedly created in a living body is automatically driven when the pressure reaches the initial siphoning pressure Ps with the siphoning minimum volume in accordance with the loop circuit in a practical clinical situation. The following are automatically repeated: the fluid starts being drained, is continuously drained, and is fully drained without remaining in the drain source and the circuit. (sustained drain necessary conditions).

However, even if

[Equation 13]

$$P - R_s > 0$$

is satisfied, in a non-siphoning circuit in which a fluid does not fill the circuit to cause a gap and drips, clogging is formed even with a slight second height difference, causing an airlocked state and disabling sustained drain. For this reason, it is required to design, in advance, the diameter and length of the drain tube in which siphoning occurs without causing a gap in accordance with the specific gravity (density) and consistency (viscosity) of the fluid when the drain circuit starts initial siphoning under the clinical loop model conditions and, as required, control also the coefficient of friction of the tube.

It is required to establish a siphoning check specification test for actually performing siphoning and checking complete draining. A circuit which has cleared the specification test is quality-assured as a sustained drain system circuit, which is assured by the quality control system for the sustained drain system circuit which controls its limitation and further improvement in quality.

A second aspect of the present invention is directed to clinical loop model conditions.

Environmental situations and conditions in use at actual practical clinical situations (such as medical practice, caring practice, and daily living sites) have not been considered at all so far, and insufficient understanding and recognition for actual use have been present. These conditions have been certified and used as being left scientifically not verified realistically. Here, the use conditions, which are major premises of the present invention, are modeled in accordance with clinical practice, a practical clinical loop model is defined, and quality is controlled under these conditions, and specifications of the product are checked so that the product can be used with security and safety also in clinical practice. In this manner, the user conditions are determined in the present invention.

That is, as a use condition in actual clinical and caring practice, the circuit has a portion with a first height difference (loop) higher than the drain source, and thereafter also has height differences. Under this condition, it is required to continuously drain. In the present system circuit model, a portion higher than the drain source is defined as a first height difference (loop) and, furthermore, each of several height differences thereafter is defined as a second height difference (loop). This clinical use environment condition is defined as a clinical loop model condition.

The first height difference is a height difference higher than the drain source, and the second height differences are several height differences occurring midway through the circuit between the drain source and the drain destination. In clinical practice, it is often the case that a height difference occurs at a portion midway through the circuit and that portion is lower than an outlet of the drain destination. In the sustained drain system circuit according to the first aspect, even with these height differences, once the circuit receives the siphoning pressure and causes siphoning, drain continues to end for complete draining.

(clinical loop model condition: details of height differences)

For the meantime, a basic specification is determined as follows: the first height difference and the second height difference are set at 20 cm and, even when the siphoning minimum volume is 50 ml, once the initial siphoning pressure is applied, drain starts and continues and complete draining is made. As required, product specifications and the conditions of the clinical loop model can be changed.

Furthermore, usage can be made for each different target by setting, in general, Vmin=50 ml for the postoperative oliguric phase, Vmin=100 ml for children, and Vmin=200 ml for adults.

Still further, for rehabilitation of the urinary bladder and the whole-body condition, standardized products with Vmin=60, 70, 80, 90 ml, or the like can be used for practical clinical rehabilitation of the functions of the urinary bladder.

A third aspect of the present invention is directed to initial siphoning pressure.

The initial siphoning pressured has been found. The driving pressure P for sustained drain is a total of the pressure Pc by pressure occurring because the elastic closed space is filled with the fluid and abdominal pressure and the pressure Pp occurring by the mass of the fluid and its potential energy.

That is, if the tip of the circuit for the fluid to flow from the drain source and the drain destination are closed, drain is impossible irrespective of whether the fluid is liquid or gas. For drain, the drain destination is required to have a structure open to atmosphere. Thus, the system circuit has a structure provided with a closed retention bag equipped with an open-ended drip chamber to make peripheral (terminal) resistance 0 (zero).

Furthermore, even if the drain destination is an open-ended circuit but the circuit has a portion higher than the drain source, when the fluid flows, the height difference resistance Rh formed of that height difference h and specific gravity (density) $\sigma$ of the fluid and, furthermore, a total resistance of the pipeline resistance Rg of the drain guide material and the pipeline resistance Rt of the drain tube are assumed. However, at the time of actual siphoning, there is a siphoning boundary where the pressure gets over the height difference h to cause the fluid to start falling down. When siphoning with the fluid filling the drain guide material and the drain tube without a gap occurs by the initial siphoning pressure with which the fluid gets over that boundary in the least to start falling down, the flow occurs without interruption, and drain continues for complete draining.

The circuit up to the siphoning boundary is occupied by part of the drain guide material and the drain tube in a clinical situation. When the volume of the former is Vhg and the height difference resistance is Rhg, the relevant components of the drain tube are denoted as Vht and Rht, respectively, in a similar manner. That is, the total volume of the circuit up to the siphoning boundary is

[Equation 14]

$$V loop = V h g + V h t$$

,

and the siphoning height difference resistance is "Equation 3 (Rh=Rhg+Rht)". On the other hand, the pipeline resistance up to the siphoning boundary is

[Equation 15]

$$R loop = f ( L hg,rg) + f (Lhr,rt)$$

,

and the initial siphoning resistance Rs=height difference resistance+pipeline resistance "Equation 8 (Rs=Rh+Rloop=Rhg+Rht+f(Lhg, rg)+f(Lht, rt)>Rg+Rt+Rp)". Then, once the pressure clears initial siphoning resistance Rs, the entire circuit thereafter becomes filled with the fluid. The siphoned circuit causes the fluid to be continuously drained, and is fully drained without the fluid retained midway. As described above, the siphoning circuit in which the pipeline resistance of the entire circuit "Equation 6 (Rall=Rg+Rt+Rp)" is decreased as much as possible to clear the initial siphoning resistance is the sustained drain system circuit according to the first or second aspect (siphoning resistance).

If knowing the meaning of

[Equation 16]

$$P = Pc + Pp$$

and understanding the principle of this system, even the user himself/herself, carer, or nurse can adjust and manage the system depending on the user's condition [management of the driving pressure P].

A fourth aspect of the present invention is directed to height difference resistance due to the use environment and resistance due to the structure of the circuit.

The height difference resistance Rh of the loop immediately after the drain source is a resistance defined by the use conditions and environment, and is not a resistance of the circuit itself. The total pipeline resistance Rail of the circuit itself as a factor inhibiting drain in the circuit is formed of the pipeline resistance Rg of the drain guide material, the pipeline resistance Rt of the drain tube, and the peripheral resistance Rp derived from the peripheral retention bag and others, and is a total of these three resistances.

"Equation 6 (Rall=Rg+Rt+Rp)" is required to be small as much as possible. The conventional retention bag circuit (urine collection bag) is of a fully-sealed type, and produces a considerable resistance, even only the peripheral resistance. By contrast, the sustained drain system circuit of the present invention includes an open-ended drip chamber, and is open to atmosphere, thereby achieving "Equation 10 (Rp=0)" [from close type to open type].

That is, in the conventional products, the height difference resistance and the peripheral (terminal) resistance due to the use environment are not considered at all.

A fifth aspect of the present invention is directed to the minimum volume an initial siphoning pressure.

While the in vivo drain source is a closed space, this space is an elastic space. In particular, the urinary bladder is an extending/contracting organ covered with a muscle wall, and its internal pressure increases as the internal volume increases. Normally, when a urine volume of approximately 200 ml to 250 ml is retained in the urinary bladder, the internal pressure of the urinary bladder is 15 cm H2O to 20 cm H2O, an initial urge to urine is felt for the first time.

Also when a balloon indwelling catheter is indwelled, the first height difference in an actual clinical situation is 15 cm to 20 cm, an internal pressure of the drain source added with the abdominal pressure and others becomes an internal pressure (initial siphoning pressure) Ps exceeding peripheral resistance including the first height difference, and the volume of the drain source getting over the siphoning boundary to start drain is defined as the siphoning minimum volume Vmin.

The sustained drain system according to the first aspect is a system circuit in which, when the inner space volumes of the drain guide material (balloon indwelling catheter) and the drain tube are Vg and Vt,

[Equation 17]

$$Vmin \geqq Vg + Vt$$

.

(siphoning minimum volume) In consideration of a use scene, the following three values are assumed for Vmin.

For the postoperative oliguric phase Vmin=50 ml
For children Vmin=100 ml
For adults Vmin=200 ml

A sixth aspect of the present invention is directed to initial siphoning pressure and sustained drain (siphoning).

In the end, the initial siphoning pressure Ps, which is a minimum necessary pressure, is determined by the siphoning resistance Rs determined by the use environment conditions and the urine collection circuit. Once the initial driving pressure satisfies

[Equation 18]

$$P > P_s = R_s$$

,

it is not required to further pressurize thereafter, and the siphoning theory acts.

That is, the principle of sustained drain in this system is the siphoning theory, and the tube connecting the drain source and the drain destination together is required to be filled with the fluid. In the conventional product, use situations in actual medical and caring practice, for example, postoperative situations and states on the user's side, among others, the first height difference resistance Rh, is not considered at all, not causing siphoning but inviting formation of clogging or an airlocked state. Although this fact may be recognized by nurses actually involved in medical care depending on the degree of experience, how much pain the fact gives actual patients as users is not recognized and no appropriate measures are taken.

In this system circuit, if the siphoning minimum volume is retained in the urinary bladder, the fluid is naturally drained sustainedly. If the user has some physical strength and can apply an abdominal pressure, with the user himself/herself having some knowledge and contriving, drain starts with a lesser volume, with internal pressure, and with load on the body. Once siphoning occurs, the fluid in the drain source is completely drained, and no residual fluid is present. This system circuit is the sustained drain system circuit according to the fourth aspect. (automatic drain start & complete drain system)

For this purpose, to start drain, a state is required in which the tube space is filled with the fluid. If the tube length is 1.2 m or longer, which is conventional, the conventional balloon catheter is used. When the minimum volume is 50 ml, the inner diameter of the tube space is set to be 6.6 mm or smaller, that is, the conventional tube space is required to be narrowed. When the fluid is dense, the coefficient of friction of the tube is required to be further decreased.

In general, to decrease the pipeline resistance, a longer diameter of the tube is better. To achieved siphoning, however, the diameter is required to be decreased.

Thus, as another factor for decreasing the pipeline resistance, it is effective to shorten the tube length and decrease the coefficient of friction of the inner surface of the tube as much as possible to make the tube smooth as much as possible. In some cases, some contrivance is required.

For example, when urine is dense, has large density and specific gravity, and has a small urine volume because of heart failure, renal failure, dehydration, or the like or when the density and consistency of urine are high because of diabetes, nephrotic syndrome, hematuria, or the like, the pipeline resistance increases accordingly and, after all, the siphoning resistance Rs also increases and the necessary initial siphoning pressure also increases.

In consideration of use for a person in a postoperative oliguric phase or having a decreased bodily function due to a serious illness, contrivance is required, in which a specification of the siphoning minimum volume Vmin=50 ml is set, the tube length of the circuit is shortened as much as possible, and the friction resistance on the surface of the inner space is reduced (major elements for product quality are the tube diameter, the tube length, and the coefficient of friction of the tube).

A seventh aspect of the present invention is directed to an initial siphoning pressure measurement method and a simple complete drain check test.

The present invention defines two methods, an initial siphoning pressure measurement method for certifying the product by actually checking whether the product conforms to the determined specifications and a simple complete drain check test method with the minimum necessary volume 50 ml (definition of specifications and contrivance of a specification conformance test)

That is, normally, industrial products, in particular, medical devices, are properly checked also in consideration of their safety and, depending on the degree of use dangerousness, their dangerousness level is also defined. However, only the conventional indwelling bladder catheter is classified as Class II, controlled medical device, in "medical device class", but a product integrated with a urine collection bag circuit is not assigned any medical device class. Moreover, the specifications of the urine collection bag circuit as a whole are not established at all. As a matter of course, no check method, institution, or system of checking whether the product satisfies specifications is present.

The sustained drain system circuit according to the first aspect to the fourth aspect is a system circuit which defines specifications of the product and a check method therefor, and the product satisfies the specifications.

The specifications are tested in consideration of actual use situations by setting a condition with two loop models whether the first height difference and the second height difference are both 20 cm as a use condition.

Description is made below by assuming the siphoning minimum volume Vmin=50 ml for a postoperative oliguric phase, where the use condition is most severe. Two siphoning check methods are defined, that is, a method of checking a clinical loop model condition and measuring its siphoning pressure and a method of checking whether the siphoning system circuit can be driven with a minimum necessary volume and can be completely drained. Furthermore, an artificial urinary bladder for checking differences and limits depending on the density and consistency of the fluid is also defined.

If the first height difference is 20 cm, the siphoning pressure is supposed to be 20 cm water column+$\alpha$. An initial siphoning pressure measurement and check method of actually checking that initial siphoning pressure has been contrived. Furthermore, the siphoning minimum necessary volume is limited to 50 ml. When pressure is applied up to the siphoning pressure for once siphoning, even if pressurization is stopped, a check is made as to whether the fluid can be fully drained by the siphoning theory from the drain source by a simple complete drain check test method, and the method has been also contrived. The sustained drain system circuit according to the first aspect to the fourth aspect includes a function of making checks through the above-described two check test methods to clear the specifications. (loop siphoning standards, check tests)

In the latter simple complete draining check test method for checking minimum volume siphoning, at the instant when a PET bottle as a drain source is subjected to a siphoning pressure to get over a siphoning boundary for siphoning, drain continues even after pressing and pressurization are stopped, and the fluid is completely drained from the PET bottle as the drain source. The PET bottle becomes at negative pressure and deformed, and the fluid is retained in a loop with the second height difference and drain stops. An actual elastic extending/contracting space of a living body such as the urinary bladder does not become at negative pressure, is not retained in the loop with the second height difference, and is completely drained also from the drain tube.

Furthermore, under this clinical loop model condition, it is required to check whether the product can clear specifications which define, as factors other than the quality (smoothness), radius, and length of the drain tube, cases in which conditions such as the density and consistency of the fluid are changed, and it is also required to grasp limits in advance. Artificial urinary bladders required to perform the conformance test by using fluids with various densities and degrees of consistency or fluids mixed with sugar, protein, blood, and/or others include one which becomes at an internal pressure of 25 cm water column, for the meantime, when Vmin=50 ml and the internal pressure is 20+a cm water column and, furthermore, one which becomes at an internal pressure higher than the initial siphoning pressure when the volume is 100 ml, 150 ml, and 200 ml, and so forth. Depending on the use purpose, artificial urinary bladders of various specifications can be created. By checking standardized products using various artificial urinary bladders and various fluids, use conditions when they are used can be clearly defined. Limits of the density and consistency of the fluid and whether the product can be used when sugar, protein, blood, or the like is mixed can be written in advance as points to be attended to. In the sustained drain system circuit, the use conditions which clear the conformance tests as described above, the specifications, and limits are clarified, and the functions, limits, and safety are assured.

An eighth aspect of the present invention is directed to variability of the specifications and utilization for rehabilitation.

Depending on the use conditions and environment, for example, for neurogenic bladder with a decreased function of the urinary bladder, as a specification for rehabilitation, a specification finely determined from Vmin=50 ml for the oliguric phase to Vmin=60 ml, 70 ml, 80 ml, and 90 ml for gradual activation of the function of the urinary bladder is provided, the specifications are gradually upgraded depending on the physical status and the function of the urinary bladder of the user, specifications are changed by applying the siphoning theory depending on the way of use, and the initial siphoning pressure is changed gradually from a small one to a large one by contriving the way of use, thereby allowing rehabilitation of the urinary bladder and physical strength.

That is, the reference artificial urinary bladder is assumed to be designed and fabricated so as to have an allowance to be able to clear the height difference of 20 cm and the pipeline resistance and so that the internal pressure becomes 25 cm water column when it is filled with the fluid 50 ml. The artificial urinary bladder in which, when filled with the fluid of 50 ml, the internal pressure becomes 25 cm water column is formed of a main body portion which is contracted by elastic force to cause the retained fluid (urine) to be drained and a connecting portion for connecting the drain guide material. The main body portion has a closed surface, and is formed of an elastic body such as silicone rubber or isoprene rubber and is formed so as to have the closed surface having an oval-shaped section. As required, a ring or grip is integrally formed to be hanged over a drip infusion stand or the like.

In a current circuit causing an artificial anuria state, the user may be caught in a vicious cycle of anuresis and may suffer urinary tract infection and/or damage by the indwelling bladder catheter. Also, the urinary bladder may be required to be washed frequently. Furthermore, the vicious cycle of anuresis, hydronephrosis, or renal failure may occur.

The present invention opens up the possibility of a virtuous cycle and rehabilitation for a pathosis forming, so far, a vicious cycle to become only degraded.

A ninth aspect of the present invention is directed to a reference artificial urinary bladder, and a reference artificial urine and a reference concentrated urine sugar.

For a final check with the properties (such as density and consistency) of the fluid finally included, an artificial urinary bladder is required. In the present invention, technical problems for establishing the specifications of that artificial urinary bladder and measuring the internal pressure of the urinary bladder have been already solved.

That is, as for the fluid flowing through the sustained drain system circuit, various concentrations, specific gravities, and degrees of consistency can be thought in accordance with actual clinical situations. Since it is burdensome to perform inspection for each fluid one by one, standard concentrated urine and reference concentrated urine sugar as urine sugar are determined for the meantime.

This reference concentrated urine sugar is defined as containing sodium chloride and glucose twice as much as normally-used replenisher components.

That is, as the reference concentrated urine sugar,

sodium chloride of 0.35 g/dl and glucose of 5.4 g/dl are provisionally determined. Initially, water is used as a fluid to measure an initial siphoning pressure, the simple complete drain check test method is performed to see if the specifications are cleared, and then, as a loading test, this reference concentrated urine sugar is retained in a reference artificial urinary bladder (volume of 50 ml and internal pressure of 25 cm water column) to perform the simple complete drain check test method on a test urine collection circuit.

As for the material and shape of the artificial urinary bladder, a first mode of the medicine injection container (Japanese Unexamined Patent Application Publication No. 2019-76327) can be used. As for measurement of the inner pressure of the artificial urinary bladder, the urinary bladder urine dynamic state measurement device (Japanese Unexamined Patent Application Publication No. 2021-72926) has been already present and can be used.

By applying these techniques, the volume and the internal pressure are set as required, and an artificial urinary bladder with set specifications can be created. The sustained drain system circuit of the product can be used by using various fluids, and its performance can be tested and checked.

A tenth aspect of the present invention is directed to a loading test.

What is required also for final check on the SDS circuit as a quality check target by using the methods introduced so far is not only the standardized artificial urinary bladder. It is also required to assume a fluid (urine) with high concentration and consistency at the time of suffering a disease such as oligohydruria, diabetes, or nephrotic syndrome at the time of oliguria assumed in an actual clinical situation, fabricate a standardized fluid in the worst state clinically assumed, perform a final loading test, as a final conformance test of a loading test, to check whether complete draining is made in the SDS circuit of the product with the reference artificial urinary bladder and this reference artificial fluid under the clinical loop model condition, thereby assuring quality or clarifying limits.

Furthermore, the tenth aspect is directed to a quality control system for the sustained drain system circuit including the loading test.

Not only an SDS circuit is invented and found, but a quality control system for the sustained drain system circuit for quality assurance and further quality refinement and improvement is constructed.

That is, a system is constructed in which basic principles and use conditions for allowing a sustained drain system circuit to be designed are clearly defined, its specifications are established, and quality is assured and controlled by a conformance check test method. That is, specifications and procedures are defined, and methods and procedures for operation are defined. A system of setting scientific experiment condition model in consideration of practical clinical conditions and situations, making objective evaluation, and checking specifications and limits is constructed to build a base for continuous improvement. A basic system is constructed to confirm and check, without arbitrary assumption, whether the purpose is properly achieved for improvement. That is, not based on an impracticable theory with a plan just created and left, but actual use conditions are properly modeled, and experiment, verification, and assurance are performed. Elements for circulating a PDCA cycle for check, evaluation, and improvement are defined, set, and systematized.

As the system components, the siphoning theory, clinical loop model conditions, an initial siphoning pressure measurement method, a simple complete draining check test method, and a reference artificial urinary bladder and a reference concentrated urine sugar to support functions depending on the properties of the fluid, and the quality control system to verify and assure the quality is presented. That is, the sustained drain system circuit of the present invention is under control of the quality control system for the sustained drain system of the fifth aspect to the ninth aspect (loop siphoning SDS quality assurance and quality control: quality control system for the sustained drain system circuit).

That is, a system is constructed which not only simply develops a new product based on new principles but also defines its specifications, assures quality, and further controls the specifications and quality. The medical device marketers approved to sell current products should introduce this system at least on their own initiative to guarantee security and safety.

For a series of specification definition, quality assurance, control, refinement, and improvement for designing a sustained drain system circuit, clarifying principles and rules for creating the circuit and establish product specifications, performing a conformance check test by using a clinical loop model, a reference artificial urinary bladder, and a reference artificial fluid for check, and reflecting the check result on the product, use conditions, and system components for improvement, the current situations of actual use are first check. Then, a purpose of the product and an action target by principles and rules therefore and the law of nature is clarified, and the product is actually designed, actually verified, sensed, evaluated, improved, and reflected. Systems thinking including PDCA in which these activities mentioned above are repeated is visualized for quality control. This is a product quality control system. Also in this sustained drain system circuit, this product quality control system is clarified and named as a quality control system for the sustained drain system circuit.

In these sustained drain system circuit and quality control system for the sustained drain system circuit, it is possible to change, as required, the specifications of the artificial urinary bladder as a drain source, the properties of the artificial urine as a fluid, and various elements of the initial siphoning resistance such as the minimum volume Vmin, the initial siphoning resistance, the tube diameter, the length of the tube, and the material of the tube, that is, the coefficient of friction of the tube. With these various conditions and various specifications, it is possible to perform a conformance check on the sustained drain system circuit, and quality of new specifications can be assured.

There are various patents and products with the siphoning theory applied, from electric appliances such as laundry machines to various measurement devices, in a larger scale, house drainage equipment or, in a further larger scale, equipment regarding electric generation in a dam. In all fields where sustained drain necessary conditions such as the siphoning initial pressure are actually grasped and defined or, furthermore, a conformance check test of determining whether the functions and performance of the initially set specifications can be actually exerted should be set to inspect the specifications and assure quality, that is, all sustained drain system circuit (siphoning) should be quality-controlled, this quality control system for the sustained drain system circuit can be utilized for "defining specifications", "quality assurance", and "control of specifications and quality", irrespective of the size, type of business, and business condition.

The system is a siphoning-check and siphoning-quality control system which checks the function of siphoning in an aspect of actual use of siphoning.

In the conventional product, use situations in actual clinical and caring practice, for example, postoperative situations and states on the user's side are not considered at all, not causing siphoning but inviting formation of clogging or an airlocked state. Although this fact may be recognized by nurses actually involved in medical care depending on the degree of experience, how much pain the fact gives actual patients as users is not recognized and no appropriate measures are taken. Here, the present invention described above has effects as follows.

(1) The present invention can model actual use conditions, clarify necessary conditions for constructing a system circuit allowing sustained drain, construct a theory for improvement of the conventional products, determine specific conditions and specifications of the system circuit conforming to the specific conditions, review components to achieve their functions, improve the conventional products, and ensure security and safety.

(2) The structure of the present invention is not essentially different from the structure of a conventional product except the open-ended drip chamber as a special device, and requires neither another special structure nor special operation

However, the present invention does not appropriately function until the principles of nature are sufficiently understood and utilized.

Furthermore, if the principles are sufficiently understood, resistance is fully reduced by utilizing activities of daily living, and uncomfortability and inconveniences of the user can be reduced.

By properly designing three elements, that is, the inner diameter, the length, and the coefficient of friction of the drain tube, and a circuit such as a urine collection bag including an open-ended drip chamber so as to clear necessary conditions for siphoning, urination starts with contraction and relax of the urinary bladder, that is, the intrinsic urologic function of the urinary bladder (micturition reflex), and the abdominal pressure. That circuit functions as a sustained drain system, and urine is completely drained without being retained in the drain source and a portion midway through the circuit.

(3) As a result, the natural function of the urinary bladder is maintained. Also, urine drain autonomously occurs with some volume retained in the urinary bladder to increase the internal pressure. Urine is sustainedly drained, and no residual urine is present in the drain tube and the urinary bladder. Anuresis not occurs, and no back flow of urine occurs and urine flows only in a forward direction. Thus, the risk of urinary tract infection is reduced. Also, rehabilitation of the urinary bladder, which has been impossible so far, can be made.

The uncomfortable feeling and pain by pressurization of the urinary bladder in an anuria state of the patient because of urine clogging caused in the conventional circuit in which urine is retained in the postoperative acute phase can be resolved.

The burden on the nursing side regarding pain of the patient can be reduced.

An anuria state is not caused by the circuit. By appropriate urine volume measurement for each time, a postoperative urine volume can be appropriately and quickly measured. Also, wrong medical decision making (such as anuresis, oliguria, heart failure, or the like) is eliminated. The unwanted use of a cardiac stimulant and a diuretic can be reduced, and hypokalemia or the like is not inflicted by administration of these drugs, thereby improving the quality of medical care and optimizing medical care.

Urine is not excessively retained in the urinary bladder, always flows only in a forward direction, and is not retained midway, thereby reducing the risk of urinary tract infection.

A backflow of urine never occurs, a flow is always in a forward direction.

Residual urine in the urinary bladder, which can be a culture medium for bacteria entering in a reverse direction, can be prevented.

The circuit is always washed away in a forward direction, thereby preventing the user from being infected from the circuit in a reverse direction.

Conventional circuit users such as seniors requiring long-term care who had been once diagnosed as anuresis or dysuria, has continuously used the conventional circuit ever since and, consequently, becoming in a chronical dysuria condition can be saved. A vicious cycle of artificial anuresis can be broken. Various iatrogenic diseases and disorder states derived from the vicious cycle can be prevented.

(1) The user can be freed from pressure on the urinary bladder and pain.

(2) Accidents of removal of the indwelling bladder catheter by circuit users themselves such as persons suffering from dementia and seniors due to pressure and pain can be avoided. Blood circulation failure, ulcers, and necrosis of the urinary bladder wall due to, for example, pressure by the bladder catheter due to early removal, can be prevented.

(3) Normally, with the urinary bladder collecting urine and filled therewith, the stretch receptor of the urinary bladder wall is stimulated, thereby causing a micturition reflex. The urinary bladder wall is contracted, and the internal and external urethral sphincters are relaxed, thereby causing urination. In the conventional circuit, since the urinary bladder is always filled and pressurized and the urinary bladder wall is continuously stretched, a stimulus as a source of a micturition reflex is inhibited, and the natural urination mechanism becomes disabled. The present invention can contribute to functional training and rehabilitation of the urination mechanism, and recovery of the function of the urinary bladder can be achieved.

(4) The users can be freed from activity limitations.

(5) Economic burdens on the users and their families who have been already in a desperate economic situation can be reduced.

(6) Family's time and trouble and care burden regarding urination can be reduced.

For anuria states due to a central nervous disorder such as spinal cord injury, cerebral hemorrhage, or cerebral infarction, or a peripheral nervous disorder, or also for bedridden users with urinary incontinence, it is possible to allow the users to urinate with the principles of nature without putting excessive loads on the users, and the present invention can be used sustainably also for a long period of time. The present invention contributes to maintenance of the remaining functions of the urinary bladder and recovery of the functions of the urinary bladder by (3) above.

By providing an appropriate urinary tract circuit to a user laying in bed for a long period of time or a bedridden user due to various illnesses, maintenance and improvement of the renal function can be expected.

The specifications of other various siphoning-theory-applied products and commodities that have been invented so far and actually used can be reviewed, and their qualities are assured and controlled.

For example, the present invention can be applied to product development and quality control of a flow-rate adjustment valve system for hydrocephalus without a differential pressure valve and without clogging.

The marketers can reduce cost, restore and increase credibility, and exercise the social responsibility and social contribution.

Accidents due to self removal, damages of the urinary bladder, dysfunction of the urinary bladder, and renal dysfunction can be prevented.

Manufacturing cost can be reduced with a simple structure.

Secure and reliable products with the principles and mechanism based on natural science can be supplied, and their effectiveness and efficacy can be properly purported.

Convenience and usability in the field of nursing and caring can be improved, thereby reducing nursing and care burdens.

The present invention contributes to prevention of misdiagnosis and malpractice in the field of medical care and achievement of high-quality medical care.

Profits of three parties, that is, the medical device marketer, the user (in the field of medical care, nursing, and caring), and the user (actual user) can be made and provided.

The corporate mission, ideal, and vison of each company can be achieved, and governance can be exerted (social responsibility and social contribution).

From sales of things, that is, products, it is possible to achieve development and enlightenment of appropriate scientific principles and system thinking via the products in the field of medical care, nursing, caring, and others, and provide services.

As a result, company's responsibility and credibility can be firmly ensured.

Users of the conventional circuit used not only in Japan but also worldwide can be saved. Also, the present invention can provide measures to solve problems of an increasing number of spinal cord injury, urinary incontinence, and benign prostatic hypertrophy due to increasingly super-aging society in the future (burdens of medical care, nursing, and caring can be reduced or eliminated, economic burdens can be eliminated, and higher quality of life (QOL) can be ensured and maintained).

BRIEF DESCRIPTION OF THE DRAWINGS

Fig. 1 is a conceptual diagram depicting components and general outlines of a urine collection bag circuit of a conventional product;

Fig. 2 is a conceptual diagram depicting components and general outlines of a use situation of a sustained drain system according to the present invention;

Fig. 3A to Fig. 3C are conceptual diagram depicting three states until siphoning according to the present invention;

Fig. 4A to Fig. 4C depict conceptual diagram depicting sources of driving the sustained drain system according to the present invention;

Fig. 5A to Fig. 5D depict conceptual diagrams depicting differences between the sustained drain system (SDS) circuit according to the present invention and a non-SDS circuit;

Fig. 6 is a conceptual diagram depicting sustained drain driving conditions under clinical loop model conditions according to the present invention;

Fig. 7 is a conceptual diagram depicting an initial siphoning pressure measurement method according to the present invention;

Fig. 8 is a conceptual diagram depicting a simple complete drain check method according to the present invention;

Fig. 9 is a conceptual diagram depicting the onset and temporal transitions of an automatic sustained drain system according to the present invention;

Fig. 10 is a conceptual diagram depicting practical clinical applied control of the sustained drain system circuit according to the present invention;

Fig. 11 is a conceptual diagram depicting an artificial urinary bladder and an internal pressure measuring device according to the present invention; and

Fig. 12 is a conceptual diagram depicting a quality control system of the sustained drain system circuit according to the present invention.

DETAILED DESCRIPTION OF THE INVENTION

An embodiment of the present invention is described based on the drawings.

In the present embodiment, while the concept of the "sustained drain system" of the present invention is applied to urination using an indwelling bladder catheter, this is merely an example, and it goes without saying that the concept can be applied as a technique of sustainedly draining a fluid from another intracorporeal closed space as a drain source to another extracorporeal space as a drain destination.

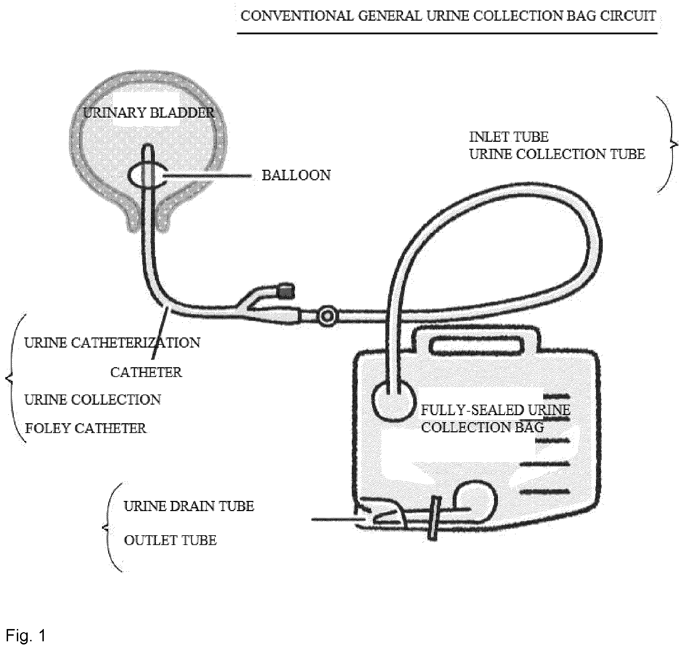

Fig. 1 depicts general outlines of a general urine collection circuit device using an indwelling bladder catheter. As depicted in the drawing, the device has a basic structure in which urine guided from an inner bladder catheter (balloon catheter) equipped with a balloon as an indwelling bladder catheter in a narrow sense is retained via a drain tube into a urine collection bag.

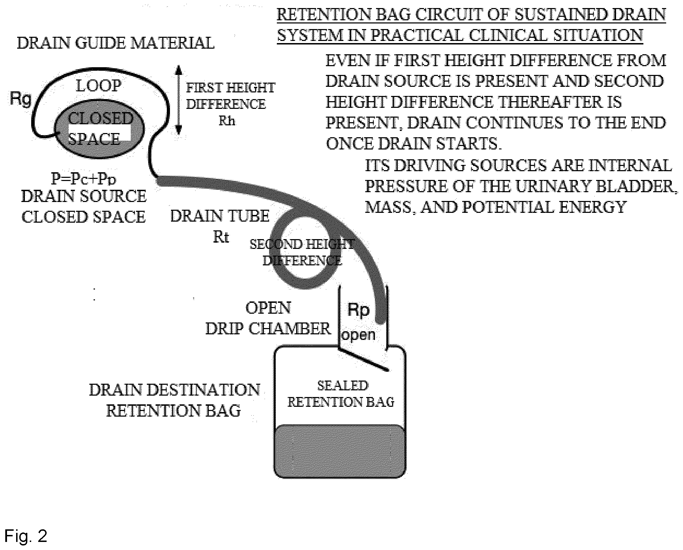

Fig. 2 depicts general outlines of the sustained drain system according to the present invention when it is assumed to be used in a practical clinical situation. The system has a basic structure in which a fluid drained from a drain source as a fluid is drained into a retention bag as a drain destination via a drain tube. However, in both clinical practice and caring practice, the drain source is not limited to be at the apex, and in most cases, a first height difference is present, in which a drain guide material or a drain tube is positioned higher than the drain source. Furthermore, several second height differences are normally present.

The drawing depicts use conditions and necessary components and structures to achieve sustained drain only in a forward direction by using scientific principles of nature even if resistance by height difference is present in consideration of the use environment in a practical clinical situation.

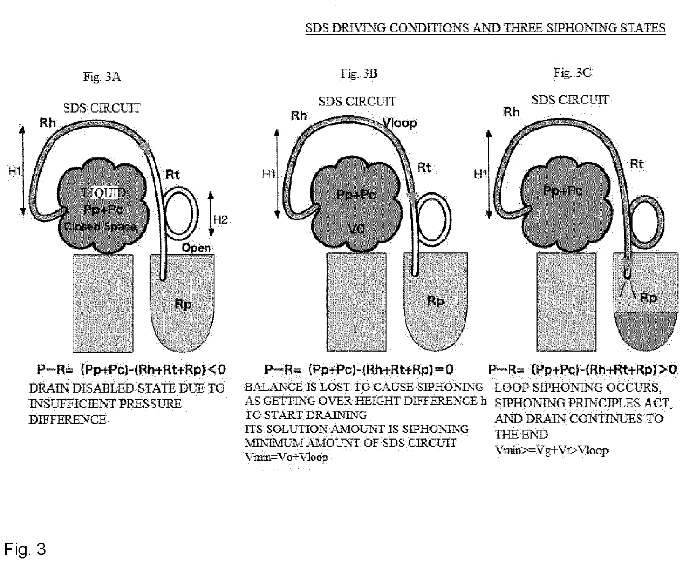

Fig. 3A to Fig. 3C depict driving conditions and their three states when this sustained drain system (SDS) circuit is connected to an elastic closed space.

The drawings depict three states until the NCSDS satisfy

[Equation 19]

$$P - R = (Pp + Pc) - (Rh + Rg + Rt + Rp) > 0$$

(In the drawings, Rg is omitted).

Fig. 3A depicts a state in which in a state of

[Equation 20]

$$P - R < 0$$

,

siphoning does not occurs and the fluid from the drain source reaches only a midway portion of the drain guide material or the drain tube and cannot be further drained.

Fig. 3B depicts an instantaneous balanced state with

[Equation 21]

$$P = R$$

in which the pressure difference and resistance are balanced and the fluid has reached a siphoning boundary of a first loop and will be drained with even a slight increase in pressure difference P or decrease in resistance R.

Here, a total of the volume of the fluid occupying the drain source (Vo) and the volume of the fluid occupying the drain guide material (omitted in the drawing) or the drain tube (Vloop) is Vmin, which is a necessary required minimum amount for driving the SDS circuit in this loop state. This is so called a minimum necessary amount for siphoning.

[Equation 22]

$$Vmin = Vo + Vloop$$

In Fig. 3C,

[Equation 23]

$$P - R > 0$$

holds, the balance is lost, and the NCSDS are satisfied to cause siphoning for sustained drain. Then, once siphoning occurs and the siphoning theory acts, the fluid of the drain source is fully drained, including the fluid at the second height difference.

That is, a feature of this SDS is that once "Equation 23 (P-R>0)" is satisfied and siphoning starts in the SDS circuit, a higher pressure is not required and drain continues.

However, the SDS is actually of no help unless Rt and Rp are minimized. That is, the SDS is not a helpful product in a practical clinical situation unless the circuit is designed in advance so that siphoning occurs even with Vmin being 50 ml or smaller and the circuit can function as an SDS circuit.

By setting siphoning minimum volume is set as small as possible, 50 ml at maximum, the SDS becomes a helpful product usable for children, people with health decline, or people with decline in the function of the urinary bladder.

For use of the conventional product of the indwelling bladder catheter of the drain guide material, it is required to review the diameter of the drain tube so as to decrease Vt so that

[Equation 24]

$$V_{min} \geqq V_g + V_t$$

.

For example, when calculation is made with a set of a balloon catheter and a drain tube indwelling bladder catheter currently commercially available,

[Equation 25]

$$V_g + V_t = \pi \times 0.25 \times 0.25 \times 40 + \pi \times 0.45 \times 0.45 \times 120 = 84.15 \, ml$$

.

Thus, when the current balloon catheter is used, to set Vmin at 50 ml, in an inverse operation, it is required to set the radius of the drain tube at 3.3 mm or smaller, that is, set the inner diameter of the drain tube at 6.6 mm or smaller.

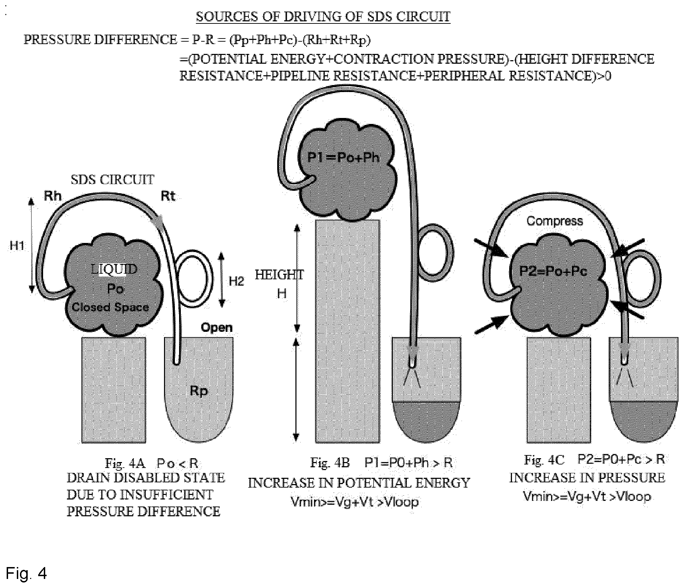

Fig. 4A to Fig. 4C are to describe the internal pressure in the elastic closed space as a source of driving this system, and depict two sources of further pressures for siphoning from a state in which pressure and resistance are balanced at the instant of siphoning.

Firstly, a method of increasing potential energy by postural change is depicted in Fig. 4B.

In an actual clinical situation, this method is relevant to a motion of sitting with knees drawn up or standing to raise the urinary bladder to prevent danger on bed where the user is laying. The inventor of the present invention has also confirmed that, at the time of a second hospital stay, in a state in which the urinary bladder was fully filled with urine even with the conventional product and it was impossible to urine although desired to do so, when the inventor stood up on bed to increase potential energy, siphoning occurred even with the conventional product for draining.

Secondly, a process of an increase in internal pressure by an increase in pressure in the closed space by external pressure (abdominal pressure) or an increase in internal pressure by an inflow of the fluid into the elastic closed space is depicted in Fig. 4C.

The two states described above, that is, increasing the internal pressure and decreasing the peripheral resistance to relatively increase pressure, are driving forces of this sustained drain system.

In any case, in practice, this does not mean that only one of these states is required. By utilizing these well, it is required to manage the internal pressure P by using the urine volume of the siphoning minimum volume Vmin and the urologic muscle of the urinary bladder and other muscles.

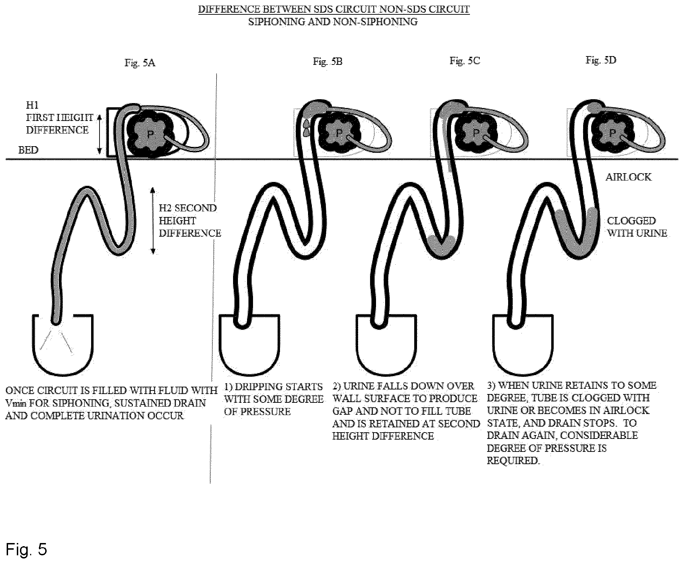

Fig. 5A to Fig. 5D depict differences between the SDS circuit and a non-SDS circuit, that is, differences between the siphoning circuit and the non-siphoning circuit.

Fig. 5A depicts a siphoned state by the SDS circuit under the use situations and conditions in a practical clinical situation already described in Fig. 3A to 3C.

In Fig. 5B onward, the drawings depict midway progress in which, since the diameter of the circuit is large, if retained urine is drained, by applying abdominal pressure to increase the internal pressure, from the inside of the urinary bladder through the drain guide material (balloon catheter) to get over the siphoning boundary to flow into the drain tube, urine does not drip and fall to fill the pipeline to cause siphoning, causing urine clogging and an airlocked state.

Since the diameter of the circuit is large, the pipeline is not filled. siphoning does not occur even the fluid gets over the first height difference and drips inside the pipeline. If this continues, as in Fig. 5C, the fluid flows down inside the drain tube and starts being retained at the second height difference. When the fluid is retained to some extent, the clogged state or the airlocked state in Fig. 5D occurs, and the fluid does not flow with the previous pressure. To cause the fluid to continuously flow, a strong pressure is required.

This problem due to the situation of the product actually used (two or more height differences) is not considered at all in the conventional products in view of the manufacturer side, the medical side, and the user side. The conventional products have been continuously used without any question in the practical clinical field and the field of caring, thereby causing artificial anuresis, which is a source of producing pseudo-oliguria and pseudo-heart failure.

If this is chronically repeated, the function of the urinary bladder and the urinary function are actually damaged and injured, thereby actually causing an anuria state. The user becomes in the anuria state not temporally but chronically, and is continuously cared with the product causing the illness. The possibility of improvement further decreases, and the user is caught in a vicious cycle and has to be cared with the conventional product in a lifetime.

However, nobody has been aware of and has pointed out this vicious cycle. The conventional products causing the users to be caught in the vicious cycle have been produced and circulated all across the world, thereby continuously producing artificial anuresis and iatrogenic diseases.

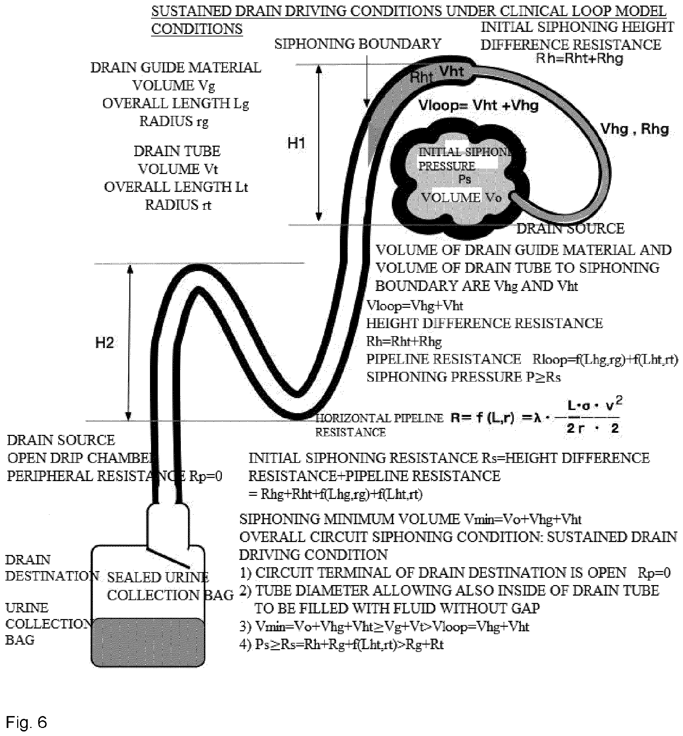

Fig. 6 depicts four necessary conditions for SDS (NCSDS) for the sustained drain system circuit of the present invention.

Instead of the conventional closed urine collection bag (retention bag), a closed urine collection bag including an open-ended drip chamber is used to eliminate terminal resistance. That is, "Equation 10 (Rp=0)", which is the "first condition".

To cause the fluid getting over the siphoning boundary to be continuously drained as directly filling the pipeline (tube) for siphoning of the entire pipeline, the diameter of the pipeline has to be small to some extent for siphoning. That is the "second condition".

The degree of appropriateness of the diameter of the drain tube depends on the clinical loop model conditions and the density and viscosity of the fluid.

For example, when the specific gravity and viscosity of urine are changed in the immediately-postoperative oliguric phase or in the dehydrated state, or due to diabetes, nephrotic syndrome, or the like, if the drain tube is too thin, it is easily clogged. Thus, the diameter is varied to be not too small and not to be large, depending on the clinical situation and conditions for use.

It is required to find one setting, to some extent, suitable for the situations and conditions as described above.

Thus, for the meantime, the height differences and the fluid for use and its volume are set as follows. That is, it is defined that the height differences are set at 20 cm, the fluid is water, and the siphoning minimum volume is 50 ml.

In a pathological state as described above, it is required to consider the necessity for appropriate resetting by adding a further change after use in an actual clinical situation.

Furthermore, siphoning is achieved by filling the inner space of the pipeline with the fluid. For this, the among of the inner space of the drain tube is required to be smaller than the siphoning minimum volume.

"Equation 24 (Vmin>Vg+Vt)" is required, and one with an indiscriminately large inner diameter and inner space of the drain tube is not suitable as an SDS, and thus the inner diameter is limited.

As the pipeline of the circuit is thinner, resistance increases. As the length of the pipeline is longer, resistance increases.

However, in a clinical situation, when a state of laying at a spine position at the center of a bed is considered, at least a half of the lateral length of the bed and a length obtained by subtracting the height of the bag from the height of the bed are required.

Also, if the length is too tight without an allowance, it is hard to move. This is not practical unless some allowance is provided, and some more space is thus required. However, this can produce a second height difference, and the pipeline may be lower than the inflow port to the urine collection bag.

In any case, even with the first height difference and the second height difference, for siphoning and sustained drain without formation of clogging, the radius r of the drain tube is defined as maximum required in accordance with the pipeline length.

For example, when a balloon catheter as a conventional product has an inner diameter of 0.5 cm and a length of 40 cm, a drain tube has a length of 120 cm, and Vmin=50 ml, in order to allow complete siphoning to be achieved, the inner diameter of the drain tube has to be set so that

[Equation 26]

$$(50-3.14 \times 0.25 \times 0.25 \times 40) \div 3.14 \div 120 = 0.11186$$

.

Its square root is 0.33 cm=3.3 mm, and the drain tube has an inner diameter of 6.6 mm or smaller. Whether siphoning with the tube space actually filled with the fluid without a gap can be achieved is unknown unless actually tried. This is a "third condition",

Lastly, the driving force of the pressure for siphoning depends on the internal pressure of the urinary bladder.

That is, it has been already described that the internal pressure is formed of two pressures, that is, the pressure Pc including the compression pressure with the urinary bladder filled with urine and the abdominal pressure and the pressure Pp by the potential energy from the mass of the fluid in the drain source (Fig. 4A to Fig. 4C).

This pressure, represented by Equation 16 (P=Pc+Pp), is larger than the siphoning resistance Rs generated by the first height difference and so forth, siphoning automatically occurs and sustained drain starts, causing complete draining. This is a "fourth condition".

Note that initial siphoning pressure=height difference resistance+pipeline resistance to siphoning boundary, and

[Equation 27]

$$Rs = Rh + Rloop = Rhg + Rht + f(Lhg, rg) + f(Lht, rt)$$

Also,

[Equation 28]

$$Vmin = Vo + Vloop = Vo + Vhg + Vht$$

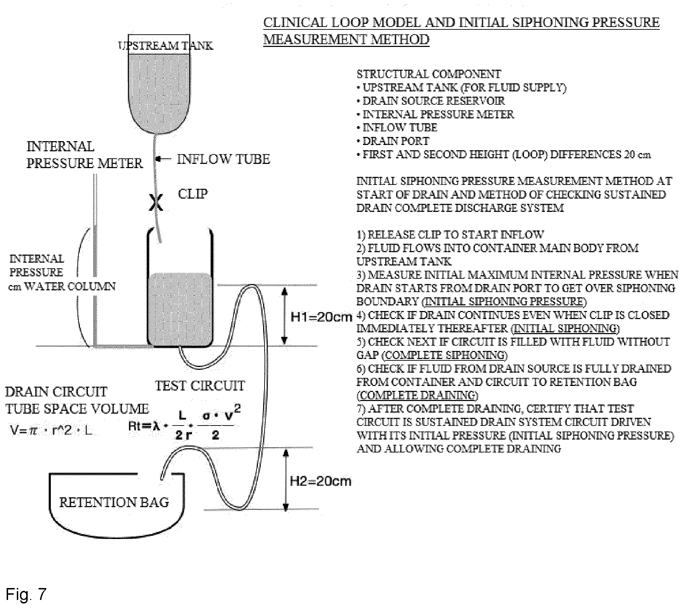

Fig. 7 is a diagram for describing an initial siphoning pressure measurement method and a method of checking a sustained drain complete drain system under clinical loop model conditions, that is, when the first and second height differences are set at 20 cm at the time of drain start.

As a matter of course, if the terminal of the circuit is completed is closed, no flow occurs at all even if a fluid is poured. Also, it is difficult to flow when the drain tube is too narrow, is bent midway, or is almost clogged.

That is, although air is also a fluid and fills the inside of the pipeline from the start, its density $\sigma$ is extremely smaller than a liquid fluid and thus the pipeline resistance by air is negligible. However, it is not negligible if the pipeline is not completely closed but is partially bent or the like to be almost closed actually.

If the pipeline is extremely bent or crushed at a certain point to have an almost closed portion, air produces resistance and even water as a liquid fluid cannot flow. Thus, the entire circuit is required to appropriately have the same diameter and the inner surface of the pipeline is also required to have uniform smoothness. If a test fluid is water, a pressure of (20+$\alpha$) cm water column obtained by the pressure at the first height difference of 20 cm water column and the pressure by pipeline pressure by the liquid fluid to the siphoning boundary is supposed to be a siphoning pressure.

When the pressure exceeds this initial siphoning pressure, siphoning occurs. Immediately thereafter, as an inflow from the upstream tank is stopped so as not to further increase the pressure and the fluid flows without an increase in pressure, the pressure is attenuated. Even so, complete siphoning occurs and drain continues, the fluid in the drain source is completely drained from the container and the circuit for complete draining. With that, complete draining by the siphoning pressure is confirmed.

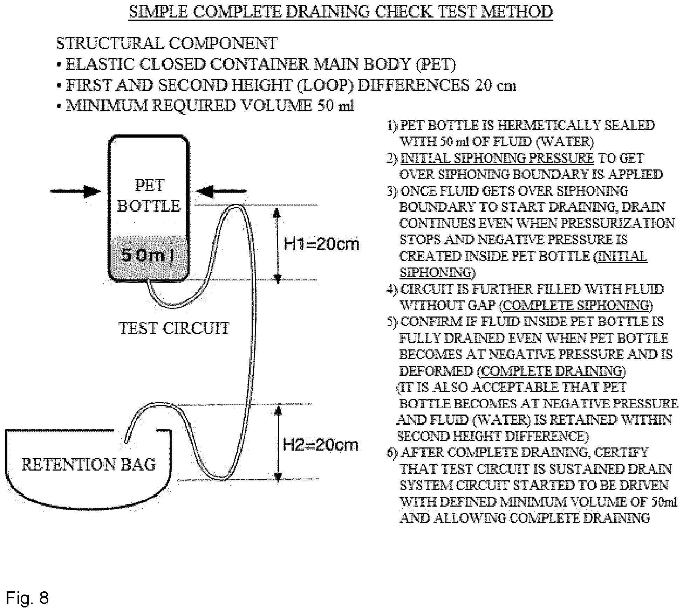

Fig. 8 depicts a simple complete drain check test method with the defined volume under similar clinical loop model conditions, which is a check test to see whether complete siphoning can be made with a fluid of 50 ml. A fluid (water) of 50 ml is poured into a PET bottle as an elastic closed space model of the drain source, and the PET bottle is hermetically sealed. The PET bottle is pressurized to the siphoning pressure measured as described above and, after initial siphoning is confirmed, complete siphoning occurs and drain continues even pressurization onto the PET bottle is stopped. The fluid is fully drained from the PET bottle, the PET bottle becomes at negative pressure to become crushed and deformed. The fluid is stopped and retained at the second height difference.

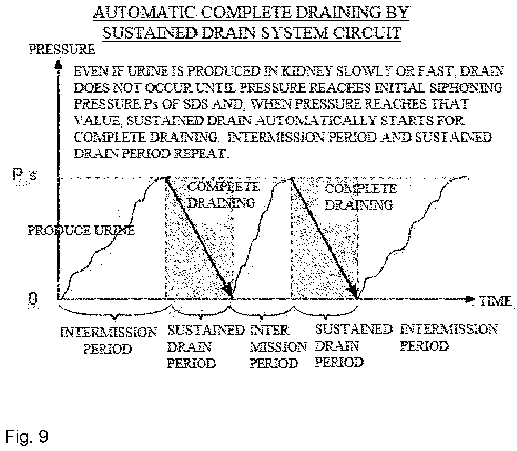

Fig. 9 schematically depicts process and progress when the sustained drain system circuit is actually used functions, in which pressure reaches the initial siphoning pressure by the urine volume retained in the urinary bladder and the abdominal pressure in a body condition at that time to automatically start drain, depicting an intermission period from production of urine until pressure reaches the initial siphoning pressure and a sustained drain period in which sustained drain starts once pressure reaches the initial siphoning pressure to cause complete draining (elimination).

One key point in the sustained drain system according to the present invention is the necessity of "understanding the urination mechanism", "design criteria of the pipeline and others for sustained drain siphoning", and "actual use situation reference".

Urine is produced in the kidney at 1 ml/Kg/h. As a urination mechanism, when 200 ml to 400 ml of urine is retained in the urinary bladder, the detrusor muscle of the urinary bladder wall is pressurized and stretched. With a stimulus by the stretch receptor, micturition reflex occurs to start urination. Here, "contraction of the detrusor muscle of the urinary bladder"+"relax of the internal urethral sphincter"+"relax of the external urethral sphincter" occurs.

Urine of 200 ml, which serves as a urination stimulus and causes a sensation of needing to urinate, is retained in the urinary bladder. Before the internal pressure of the urinary bladder increases to exceed a threshold, the urine collection circuit is required to be siphoned. However, to start drain with a urine volume as minimum as possible, the resistance Rh by the first height difference HI is not avoidable in a practical clinical use situation. Among the other three circuit resistance elements Rg, Rt, and Rp, Rg is not much allowed to be changed except the coefficient of friction, and the other resistances Rp and Rt are required to be decreased as much as possible.

Rp becomes approximately 0 as an open-ended bag in the open-ended drip chamber, and the remaining Rt is minimized as much as possible, thereby providing the function of siphoning even in a postoperative biological condition or in a wheelchair with chronic anuresis. Without this, sustained drain cannot start.

Furthermore, to measure chronological urine volume (time urine) serving as an important index of the postoperative physical state, it is required to automatically start urination even with the siphoning necessary minimum volume Vmin equal to or smaller than 50 ml.

For example, for the purpose of measurement of postoperative time urine, it is not enough to retain urine in the urinary bladder until a sensation of needing to urinate occurs with 200 ml to start urination, as is normally the case. It is required to design Vmin so that sustained drain starts with a urine volume as small as possible.

Also for siphoning, it is required to fill the urine collection circuit with a urine volume as small as possible. The most important factors for siphoning are Rh due to the presence of the first height difference HI and the pipeline resistance Rt, which are clinically required.

It is the muscle group of the urinary bladder and other muscle groups regarding the abdominal pressure and the urine volume filling the urinary bladder that produce pressure to get over the resistance. After all, also in order to decrease the siphoning minimum necessary volume Vmin as much as possible, minimizing the resistance Rt of the drain tube as much as possible is the only way.

Rh is a height difference clinically required irrespective of whether the user lays in bed or is in a wheelchair, and cannot be omitted.

However, Rh can be decreased depending on the body state, by doing something even laying in bed. This is not a normal situation, but can trigger the conscious start of urination.

After all, no other option remains but to decrease the pipeline resistance Rt as a factor for adjusting the siphoning minimum volume Vmin. The sustained drain system cannot be controlled unless designed in advance.

Vmin is a sum of the retention volume of the urinary bladder producing a SDS driving internal pressure difference and a pipeline-filled volume for getting over the first height difference.

$$\text{"Equation 28 (Vmin=Vo+Vloop=Vo+Vhg+Vht)"}$$

Pc is a contraction pressure produced by the detrusor muscle of the urinary bladder stretched with an increase in the internal pressure by retention of urine, and requires some urine volume. Also, Pp is not produced unless some urine volume is retained.

The actual volume of the urinary bladder which actually causes pressure to reach the initial siphoning pressure varies depending on the physical state and the urinary bladder state of the SDS user at that time, varying with time and individual. After all, whenever pressure reaches the initial siphoning pressure, complete draining occurs for each time in a sustained drain period in which urination automatically occurs.

The factor for defining the initial siphoning pressure is resistance. When the use of the conventional balloon catheter as a drain guide material is assumed, if urine is collected via the open-ended drip chamber, that is, "Equation 3 (Rp=0)", Rt is the only factor to be controlled, except Rh clinically required. By controlling Rt, siphoning is prepared and, in the sustained drain system, in each of physical states at different times, an appropriate volume of urine is retained in the urinary bladder to cause pressure to reach the initial siphoning pressure for automatic repetitive urinations.

Rt obtained by excluding the height difference resistance Rh, which is not an element of the circuit, from Rs is a determination factor for determining whether to start sustained drain when some volume of urine is retained. To cause sustained drain without putting a load on the living body as much as possible (to exclude the necessity of applying excessive abdominal pressure), it is required to automatically start sustained drain with minimum necessary resistance and a minimum urine volume for siphoning.

In the pipeline resistance, the density and flow velocity of the fluid are involved. However, the fluid density σ cannot be controlled because of depending on the concentration of urine produced. Moreover, the flow velocity is determined by the pipeline and the pressure difference accordingly, and also cannot be controlled. On the other hand, the inner diameter and the length of the pipeline can be determined in advance, and therefore can be controlled.

As described above, as for Rt, the radius and the length are key factors. Furthermore, in some cases, friction resistance λ and the density and consistency of the fluid may also be considered. In those cases, it is required to ensure capabilities and functions capable of draining dense urine due to dehydration, heart failure, renal failure, or the like or high-viscosity urine due to diabetes, nephrotic syndrome, or the like.

After all, the radius r and the pipeline length L of the pipeline define the resistance Rt and the sustained drain start urine volume Vmin, and also determine the start of SDS.

That is, two major factors for sustained drain are siphoning and pressure difference control. After all, as represented in Equation 11, the length and inner diameter (radius) of the circuit are important factors and it is no exaggeration to say that sustained drain is defined by the inner diameter.

The length and the inner diameter can be both designed in advance. If the inner diameter of the drain tube is 4 mm (radius of 2 mm) and the length is 1.2 m, Vt is approximately 15 ml.

Also to suppress occurrence of clogging of the drain tube with urine and airlock, siphoning with a thin pipeline is required. With siphoning and the pressure difference, complete draining can be made without residual urine.

From above,

$$\text{"Equation 12 } (P\text{-}Rs{>}0)\text{"}$$

is the driving force of the automatic drain system. Appropriate control and management cannot be performed at the time of manufacturing and nursing in charge of actual operation and management unless this concept of siphoning is sufficiently understood. Insufficient understanding, by extension, may become a major inhibiting factor when appropriate medical care is implemented.

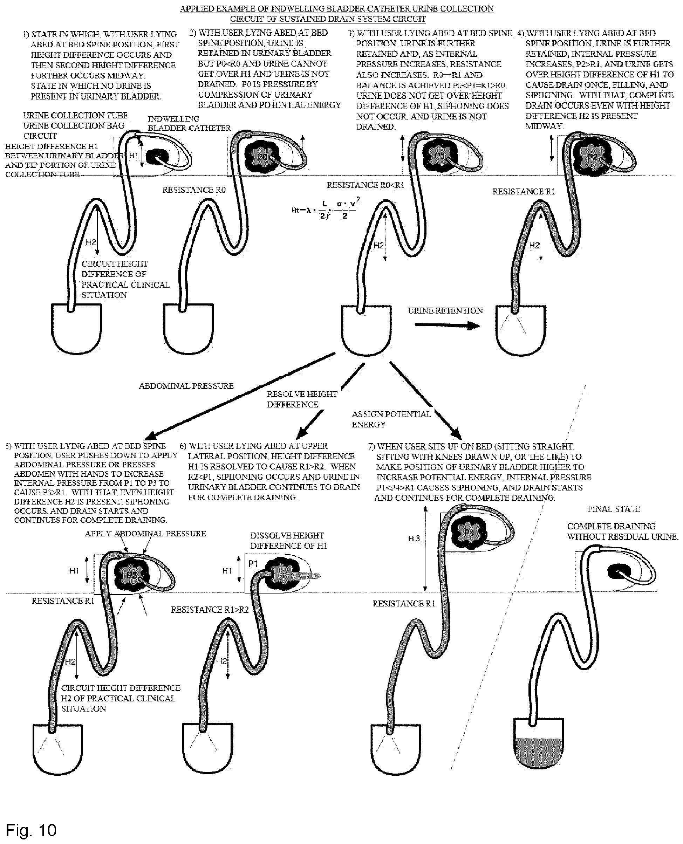

Fig. 10 introduces a method for driving this SDS in an actual clinical scene.

In (4) of Fig. 10, a case is depicted in which urine is gradually retained in the urinary bladder, and the inner pressure (P2) exceeds the total resistance (R) including the first height difference.

In (5) of Fig. 10, a case is depicted in which the user applies abdominal pressure by himself/herself by pushing down, pressing the abdomen with hands, or the like.

In (6) of Fig. 10, a case is depicted in which the user changes the posture from a spine position to a lateral position to resolve the first height difference to decrease the siphoning resistance Rs.

In (7) of Fig. 10, a case is depicted in which the potential energy is increased by changing the posture from a spine position to a position with knees drawn up or a standing position.

In (8) of Fig. 10, a case is depicted in which, as a result, complete urination has been made.

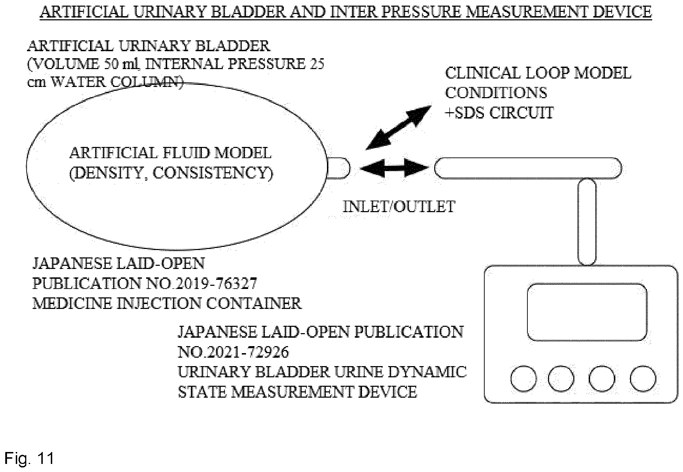

Fig. 11 depicts the structure and functions of a reference urinary bladder required for the simple complete draining check test method to check whether the product conforms to the specifications and also for a loading test with the properties of a concentrated thick fluid, and also depicts a urinary bladder urine dynamic state measurement device to measure the pressure. By using the artificial urinary bladder in place of a simple PET bottle, complete discharge can be made without retention and stagnation in the second loop.

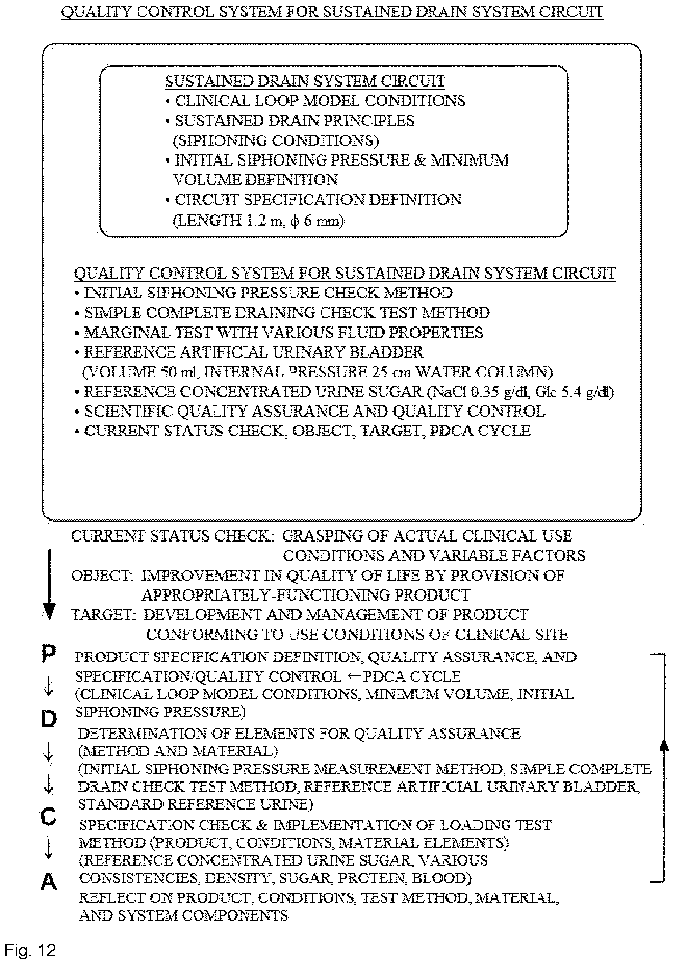

Fig. 12 depicts necessary elements when the sustained drain system circuit is fabricated, and necessary elements for planning its specifications and assuring its quality, and an overview of a quality assurance and quality control system with PDCA applied to fabricate proper medical devices and control their quality.

With this sustained drain control system, the sustained drain system circuit is properly fabricated and properly used and operated, thereby enhancing convenience in clinical control, performing proper medical care, and improving the quality of life of the users and their families.

Also this reason, the reference artificial urinary bladder, the reference concentrated urine sugar, and the clinical loop model conditions are required to be studied. With the current circumstances accurately confirmed to serve as a base of the study, it is imperative to perform systems thinking of the purpose and target and the PDCA cycle.

In the sustained drain system of the present invention, by further contriving a urine collection tube as a drain tube in view of its material to reduce its inner diameter, length, and the coefficient of friction in consideration of the use conditions and environment, the initial siphoning resistance can be reduced, and there is a possibility of reducing the load on the urinary bladder and the body. In other words, this SDS circuit depends on how this drain tube is contrived so as to have small resistance and be light in weight and usable with ease in a clinical situation. For this purpose, by utilizing patents and know-hows regarding artificial vessels, at least low-quality tubes clogged with bloody urine do not have to be provided. That is, an ideal circuit is born like an artificial vessel almost without friction resistance and not clogged with viscous concentrated urine, urine sugar, urine protein, bloody urine, or the like even the circuit is thin to allow sustained drain.

Furthermore, the system can be used not only for urination using an indwelling bladder catheter but can be widely used as a principle and technique for sustainedly draining a fluid from another intracorporeal elastic closed space as a drain source to an extracorporeal space as a drain destination.

These principles of siphoning and system circuit have the possibility of helping construction of not only a urine collection circuit but also a catheter for discharging a postoperative waste fluid such as a pleural fluid or ascites fluid and a shunt system from the cerebral ventricle to the thoracic cavity or the abdominal cavity for treatment of hydrocephalus. A system circuit in which a certain resistance is established in advance by the length, radius, and the friction resistance in accordance with the use purpose can be used as a shunt system for hydrocephalus which automatically cause sustained drain once a certain pressure difference occurs, even if a mechanical system is not present, such as a flow-rate adjustment valve (differential pressure valve) from the cerebral ventricle to the inside of the thoracic cavity or the abdominal cavity in one direction. As the number of mechanical portions increases, the number of causes of clogging or failure increases. With the same principles, it is possible to construct a system only with a circuit, the system in which a flow does not occur at a spine position but automatic sustained drain occurs when a pressure difference occurs between the pressure in the cerebral ventricle and the pressure in the thoracic cavity or the abdominal cavity at a sitting position or a standing position.

Furthermore, in addition to the system circuit, the quality control system of the present invention according to the first aspect to the tenth aspect in Fig. 12 provides a procedure and method of analyzing use conditions in an actual use scene to provide a properly-functioning product, not limited to a medical device, in any product development, and performing product development and control conforming to the use conditions. These procedure and method are useful for any company and any product. Even without official specifications, the company itself can perform quality assurance and quality control of the product in the name of the company and can exert its existence value and accomplish a mission of the company.

The conventional products are deviated to disregard the current situations already at the first step of this control system of grasping the use conditions for checking the current state and variable factors. That is, use situations in actual medical and caring practice, for example, postoperative situations and states on the user's side, among others, the influence of the first height difference, is not considered at all, not causing siphoning but inviting formation of clogging or an airlocked state. Although this fact may be recognized by nurses actually involved in medical care depending on the degree of experience, the current circumstances are such that how much pain the fact gives actual patients as users is not recognized and no appropriate measures are taken.

Thus, when it is defined that a pressure difference between the drain source and the drain destination is P, resistance of the fluid with respect to drain is R, a minimum volume as small as possible satisfying the elastic closed space as a drain source necessary for producing the siphoning pressure Ps as an initial pressure difference for siphoning which gets over the siphoning resistance Rs formed of the height difference resistance of the first height difference occurring by an initial loop of the loop model conditions in consideration of actual use environment conditions to start sustained drain is Vmin, and a volume occupying the tube space of the first height difference of the siphoning boundary where the pressure gets over the peak of the first height difference with the siphoning pressure Ps to start drain is Vloop, a relation between the inner space volume Vg of the drain guide material and the inner space volume Vt of the drain tube midway in the circuit is represented as

[Equation 29]

$$\mathrm{V\,min} \geqq \mathrm{V\,g} + \mathrm{V\,t} > \mathrm{Vloop}$$

.

The siphoning circuit is provided so that the above equation is satisfied and the inner diameter of the tube space is designed so that the fluid is drained as filling the tube space without a gap even during draining. Once the pressure difference P exceeds the Ps value balanced with the siphoning resistance Rs and satisfying "Equation 11 (Ps≥Rs=Rh+Rg+f(Lht, rt)>Rg+Rt), siphoning occurs and, even with a second height difference occurring due to a loop after the first height difference, a negative pressure is not formed and drain continues by elasticity of the drain source for complete draining.

For this purpose, in the sustained drain system circuit, actual use situations and environments are modeled, and circuit conditions for siphoning under these conditions are clarified and properly defined, specification are further defined, and quality is assured. Also, a quality control system for the sustained drain system circuit is constructed, a loading test is also performed by the quality control system, and the sustained drain system circuit is a product assured with its specifications and limitations clarified. The quality control system includes three processes of "definition of specifications", "quality assurance", and "specification and quality control", and circulates these processes.

**Claims**

1. A sustained drain system circuit in which a fluid is caused to be sustainedly drained by siphoning from an intracorporeal elastic closed space that can extend and contract as a drain source, comprising:

   a drain guide material provided to cause the fluid to be drained from the drain source;
   a drain tube for moving the fluid from the drain guide material to a drain destination; and
   a closed retention bag as the drain destination including an open-ended drip chamber, wherein
   when a pressure difference between the drain source and the drain destination is P and a pipeline resistance of the circuit as a whole with respect to drain of the fluid is Rail,
   a specified minimum volume to produce an initial siphoning pressure Ps as an initial pressure difference for siphoning to get over an initial siphoning resistance Rs due to a first height difference HI in a clinical loop model condition in consideration of an actual use environment condition and the pipeline resistance to start sustained drain is Vmin,
   a volume in Vmin filling the elastic closed space is Vo, a volume occupying a tube space of the first height difference to a siphoning boundary where the initial siphoning pressure Ps gets over a peak of the first height difference to start drain is Vloop, an inner space amount of the drain guide material in Vloop is Vhg, an inner space amount of the drain tube in Vloop is Vht and, furthermore, a total inner space volume of the drain guide material is Vg and a total inner space volume of the drain tube is Vt, a relation is represented by

   [Equation 1]

   $$V\,min = V\,o + V\,h\,g + V\,h\,t \geqq Vg + Vt > Vloop = V\,h\,g + V\,h\,t$$
   ,

   and the circuit is a siphoning circuit in which an inner diameter of a pipe space is designed so that a siphoning volume condition defined by Equation 1 is satisfied and the fluid is drained as filling without a gap also when being drained inside the pipe space; siphoning occurs once the pressure difference P exceeds Ps balanced with the initial siphoning resistance Rs; and, even if a second height difference H2 occurring due to a loop after the first height difference HI is present, drain continues without creating negative pressure by elasticity of the drain source and complete draining is made.

2. The sustained drain system circuit according to claim 1, wherein
   the first height difference is a height difference necessary for a practical clinical situation and higher than the drain source by the loop, the second height difference is one or more height differences occurring by the loop after the first height difference and midway in the circuit between the drain source and the drain destination and, even if the first height difference and the second height differences are present, once the circuit receives the initial siphoning pressure Ps to become siphoning, drain continues to end for complete draining.

3. The sustained drain system circuit according to claim 2, wherein
   when a height difference resistance produced from the first height difference H1 and a specific gravity (density) σ of the fluid from the drain source to the siphoning boundary is Rh; a height difference resistance of the drain guide material in Rh is Rhg, a height difference resistance Rht of the drain tube to the siphoning boundary is Rht, and a total of Rhg and Rht is the height difference resistance Rh; a pipeline resistance of an overall length of the drain guide material is Rg, a pipeline resistance of an overall length of the drain tube is Rt, and a resistance at the drain destination is a peripheral resistance Rp; and, in general, a length of the circuit is L, a circuit inner diameter is 2r, a flowing speed is v, and a coefficient of friction of the pipeline is λ, a pipeline resistance is represented as

   [Equation 2]

   $$R = f\,(\,\lambda, L, \sigma, v, r) = f\,(L, r) = \lambda \cdot\ L \cdot \sigma \cdot\ v^2 / 4r$$
   ,

   when a radius of the drain guide material is rg, a radius of the drain tube is rt, a circuit length of the drain guide material to the siphoning boundary is Lhg, a circuit length of the drain tube to the siphoning boundary is Lht, an overall length of the drain guide material is Lg, and an overall length of the drain tube is Lt, a height difference resistance to the siphoning boundary by a use condition is represented as

[Equation 3]

$$Rh = Rhg + Rht$$

,

an actual pipeline resistance to the siphoning boundary is

[Equation 4]

$$Rloop = f(Lhg,rg) + f(Lht,rt)$$

,

the initial siphoning resistance Rs is a total of Rh and Rloop, that is,

[Equation 5]

$$Rs = Rh + Rloop$$

,

the pipeline resistance Rall of the system circuit as a whole is obtained by minimizing

[Equation 6]

$$Rall = Rg + Rt + Rp$$

,

siphoning occurs when

[Equation 7]

$$Ps \geqq Rs > Rall = Rg + Rt + Rp$$

is satisfied,

[Equation 8]

$$Rs = Rh + Rloop = Rhg + Rht + f(Lhg,rg) + f(Lht,rt) > Rg + Rt + Rp$$

is satisfied and, furthermore, if the siphoning boundary is the drain tube,

[Equation 9]

$$f(Lhg, rg) = Rg$$

holds, By using the open-ended drip chamber, the system circuit is set so that

[Equation 10]

$$Rp = 0$$

,

thus,

[Equation 11]

$$Ps \geqq Rs = Rh + Rg + f(Lht, rt) > Rg + Rt$$

is satisfied.

4. The sustained drain system circuit according to claim 2, wherein
the pressure difference P as a driving force of the circuit is formed of a compression pressure Pc formed of a compression pressure occurring by the fluid being retained in the elastic closed space or an internal pressure in an abdominal cavity or pleural cavity in a living body and a pressure Pp of a weight by potential energy of a mass of the fluid retained in the elastic closed space.

5. A quality control system for the sustained drain system circuit according to claim 2, wherein the sustained drain system circuit is certified by a conformance test by an initial siphoning pressure measurement method and a simple complete draining check test method as specification conformance check methods for the sustained drain system circuit, the methods in which the sustained drain system circuit is checked under the clinical loop model condition whether the sustained drain system circuit is a product actually driven with the specified and set initial siphoning pressure and the minimum volume and also the initial siphoning pressure is actually measured and checked.

6. The quality control system for the sustained drain system circuit according to claim 5, wherein whether the sustained drain system circuit is driven by the fluid with a condition reflecting a clinical condition is controlled by a PDCA cycle including a reference loading test using a reference artificial urinary bladder and a reference concentrated urine sugar and a loading marginal check method to check a limit.

7. The quality control system for the sustained drain system circuit according to claim 6, wherein the reference artificial urinary bladder is designed and fabricated so as to have an allowance to clear a height difference of 20 cm and the pipeline resistance and so as to have an internal pressure of 25 cm water column when filled with 50 ml of the fluid.

8. The quality control system for the sustained drain system circuit according to claim 6, wherein as the fluid flowing through the sustained drain system circuit, reference artificial urine, the reference concentrated urine sugar, and other artificial urine in a clinical situation are subjected to methods including a specification conformance check test, a loading test, and the loading marginal check method to check the limit in the initial siphoning pressure measurement method and the simple complete draining check test method.

9. The quality control system for the sustained drain system circuit according to claim 6, wherein
   factors for the initial siphoning resistance including specifications of the reference artificial urinary bladder as the drain source, properties of the reference concentrated urine sugar as the fluid, the specified minimum volume Vmin, and a diameter, length, material, and coefficient of friction of the pipeline can be changed as required, and a conformance check to see whether the sustained drain system circuit is relevant can be made also with various conditions and specifications for quality assurance.

10. The sustained drain system circuit according to claim 1, wherein
    also in a situation and environment in actual use practice, a conformance check test is performed by the quality control system for the sustained drain system circuit according to claim 6 as to whether siphoning is actually made and sustained drain is possible, and specifications are verified to assure quality.

CONVENTIONAL GENERAL URINE COLLECTION BAG CIRCUIT

URINARY BLADDER

INLET TUBE
URINE COLLECTION TUBE

BALLOON

URINE CATHETERIZATION
CATHETER
URINE COLLECTION
FOLEY CATHETER

FULLY-SEALED URINE
COLLECTION BAG

URINE DRAIN TUBE
OUTLET TUBE

Fig. 1

DRAIN GUIDE MATERIAL

RETENTION BAG CIRCUIT OF SUSTAINED DRAIN
SYSTEM IN PRACTICAL CLINICAL SITUATION

EVEN IF FIRST HEIGHT DIFFERENCE FROM
DRAIN SOURCE IS PRESENT AND SECOND
HEIGHT DIFFERENCE THEREAFTER IS
PRESENT, DRAIN CONTINUES TO THE END
ONCE DRAIN STARTS.

ITS DRIVING SOURCES ARE INTERNAL
PRESSURE OF THE URINARY BLADDER,
MASS, AND POTENTIAL ENERGY

LOOP

Rg

CLOSED SPACE

FIRST HEIGHT DIFFERENCE
Rh

$P=P_C+P_D$
DRAIN SOURCE
CLOSED SPACE

DRAIN TUBE
Rt

SECOND HEIGHT DIFFERENCE

OPEN
DRIP CHAMBER

Rp
open

DRAIN DESTINATION
RETENTION BAG

SEALED
RETENTION BAG

Fig. 2

SDS DRIVING CONDITIONS AND THREE SIPHONING STATES

Fig. 3A

SDS CIRCUIT

$P-R= (Pp+Pc)-(Rh+Rt+Rp)<0$

DRAIN DISABLED STATE DUE TO
INSUFFICIENT PRESSURE
DIFFERENCE

Fig. 3B

SDS CIRCUIT

$P-R= (Pp+Pc)-(Rh+Rt+Rp)=0$

BALANCE IS LOST TO CAUSE SIPHONING
AS GETTING OVER HEIGHT DIFFERENCE h
TO START DRAINING
ITS SOLUTION AMOUNT IS SIPHONING
MINIMUM AMOUNT OF SDS CIRCUIT
Vmin=Vo+Vloop

Fig. 3C

SDS CIRCUIT

$P-R= (Pp+Pc)-(Rh+Rt+Rp)>0$

LOOP SIPHONING OCCURS,
SIPHONING PRINCIPLES ACT,
AND DRAIN CONTINUES TO
THE END
Vmin>=Vg+Vt>Vloop

Fig. 3

EP 4 190 371 A1

SOURCES OF DRIVING OF SDS CIRCUIT

PRESSURE DIFFERENCE = P-R = (Pp+Ph+Pc)-(Rh+Rt+Rp)
= (POTENTIAL ENERGY+CONTRACTION PRESSURE)-(HEIGHT DIFFERENCE RESISTANCE+PIPELINE RESISTANCE+PERIPHERAL RESISTANCE)>0

Fig. 4A   Po < R
DRAIN DISABLED STATE
DUE TO INSUFFICIENT
PRESSURE DIFFERENCE

Fig. 4B   P1=P0+Ph > R
INCREASE IN POTENTIAL ENERGY
Vmin>=Vg+Vt >Vloop

Fig. 4C   P2=P0+Pc > R
INCREASE IN PRESSURE
Vmin>=Vg+Vt >Vloop

Fig. 4

29

DIFFERENCE BETWEEN SDS CIRCUIT NON-SDS CIRCUIT
SIPHONING AND NON-SIPHONING

Fig. 5A          Fig. 5B          Fig. 5C          Fig. 5D

H1
FIRST HEIGHT
DIFFERENCE

BED

AIRLOCK

H2 SECOND
HEIGHT
DIFFERENCE

CLOGGED
WITH URINE

ONCE CIRCUIT IS FILLED WITH FLUID WITH
Vmin FOR SIPHONING, SUSTAINED DRAIN
AND COMPLETE URINATION OCCUR

1) DRIPPING STARTS
WITH SOME DEGREE
OF PRESSURE

2) URINE FALLS DOWN OVER
WALL SURFACE TO PRODUCE
GAP AND NOT TO FILL TUBE
AND IS RETAINED AT SECOND
HEIGHT DIFFERENCE

3) WHEN URINE RETAINS TO SOME
DEGREE, TUBE IS CLOGGED WITH
URINE OR BECOMES IN AIRLOCK
STATE, AND DRAIN STOPS. TO
DRAIN AGAIN, CONSIDERABLE
DEGREE OF PRESSURE IS
REQUIRED.

Fig. 5

SUSTAINED DRAIN DRIVING CONDITIONS UNDER CLINICAL LOOP MODEL CONDITIONS

Fig. 6

CLINICAL LOOP MODEL AND INITIAL SIPHONING PRESSURE MEASUREMENT METHOD

STRUCTURAL COMPONENT
• UPSTREAM TANK (FOR FLUID SUPPLY)
• DRAIN SOURCE RESERVOIR
• INTERNAL PRESSURE METER
• INFLOW TUBE
• DRAIN PORT
• FIRST AND SECOND HEIGHT (LOOP) DIFFERENCES 20 cm

INITIAL SIPHONING PRESSURE MEASUREMENT METHOD AT START OF DRAIN AND METHOD OF CHECKING SUSTAINED DRAIN COMPLETE DISCHARGE SYSTEM

1) RELEASE CLIP TO START INFLOW
2) FLUID FLOWS INTO CONTAINER MAIN BODY FROM UPSTREAM TANK
3) MEASURE INITIAL MAXIMUM INTERNAL PRESSURE WHEN DRAIN STARTS FROM DRAIN PORT TO GET OVER SIPHONING BOUNDARY (INITIAL SIPHONING PRESSURE)
4) CHECK IF DRAIN CONTINUES EVEN WHEN CLIP IS CLOSED IMMEDIATELY THEREAFTER (INITIAL SIPHONING)
5) CHECK NEXT IF CIRCUIT IS FILLED WITH FLUID WITHOUT GAP (COMPLETE SIPHONING)
6) CHECK IF FLUID FROM DRAIN SOURCE IS FULLY DRAINED FROM CONTAINER AND CIRCUIT TO RETENTION BAG (COMPLETE DRAINING)
7) AFTER COMPLETE DRAINING, CERTIFY THAT TEST CIRCUIT IS SUSTAINED DRAIN SYSTEM CIRCUIT DRIVEN WITH ITS INITIAL PRESSURE (INITIAL SIPHONING PRESSURE) AND ALLOWING COMPLETE DRAINING

UPSTREAM TANK

INTERNAL PRESSURE METER ← INFLOW TUBE

CLIP

INTERNAL PRESSURE cm WATER COLUMN

H1=20cm

DRAIN CIRCUIT TUBE SPACE VOLUME

TEST CIRCUIT

$V = \pi \cdot r^2 \cdot L$

$Rt = \lambda \cdot \dfrac{L}{2\,r} \cdot \dfrac{\sigma \cdot v^2}{2}$

H2=20cm

RETENTION BAG

Fig. 7

## SIMPLE COMPLETE DRAINING CHECK TEST METHOD

STRUCTURAL COMPONENT
• ELASTIC CLOSED CONTAINER MAIN BODY (PET)
• FIRST AND SECOND HEIGHT (LOOP) DIFFERENCES 20 cm
• MINIMUM REQUIRED VOLUME 50 ml

PET BOTTLE

5 0 m l

H1=20cm

TEST CIRCUIT

RETENTION BAG

H2=20cm

1) PET BOTTLE IS HERMETICALLY SEALED
WITH 50 ml OF FLUID (WATER)
2) INITIAL SIPHONING PRESSURE TO GET
OVER SIPHONING BOUNDARY IS APPLIED
3) ONCE FLUID GETS OVER SIPHONING
BOUNDARY TO START DRAINING, DRAIN
CONTINUES EVEN WHEN PRESSURIZATION
STOPS AND NEGATIVE PRESSURE IS
CREATED INSIDE PET BOTTLE (INITIAL
SIPHONING)
4) CIRCUIT IS FURTHER FILLED WITH FLUID
WITHOUT GAP (COMPLETE SIPHONING)
5) CONFIRM IF FLUID INSIDE PET BOTTLE IS
FULLY DRAINED EVEN WHEN PET BOTTLE
BECOMES AT NEGATIVE PRESSURE AND IS
DEFORMED (COMPLETE DRAINING)
(IT IS ALSO ACCEPTABLE THAT PET
BOTTLE BECOMES AT NEGATIVE PRESSURE
AND FLUID (WATER) IS RETAINED WITHIN
SECOND HEIGHT DIFFERENCE)
6) AFTER COMPLETE DRAINING, CERTIFY
THAT TEST CIRCUIT IS SUSTAINED DRAIN
SYSTEM CIRCUIT STARTED TO BE DRIVEN
WITH DEFINED MINIMUM VOLUME OF 50ml
AND ALLOWING COMPLETE DRAINING

Fig. 8

AUTOMATIC COMPLETE DRAINING BY
SUSTAINED DRAIN SYSTEM CIRCUIT

PRESSURE

EVEN IF URINE IS PRODUCED IN KIDNEY SLOWLY OR FAST, DRAIN
DOES NOT OCCUR UNTIL PRESSURE REACHES INITIAL SIPHONING
PRESSURE Ps OF SDS AND, WHEN PRESSURE REACHES THAT
VALUE, SUSTAINED DRAIN AUTOMATICALLY STARTS FOR
COMPLETE DRAINING. INTERMISSION PERIOD AND SUSTAINED
DRAIN PERIOD REPEAT.

P s

COMPLETE
DRAINING

COMPLETE
DRAINING

PRODUCE URINE

0

TIME

INTERMISSION
PERIOD

SUSTAINED
DRAIN
PERIOD

INTER
MISSION
PERIOD

SUSTAINED
DRAIN
PERIOD

INTERMISSION
PERIOD

Fig. 9

Fig. 10

ARTIFICIAL URINARY BLADDER AND INTER PRESSURE MEASUREMENT DEVICE

ARTIFICIAL URINARY BLADDER
(VOLUME 50 ml, INTERNAL PRESSURE 25
cm WATER COLUMN)

CLINICAL LOOP MODEL
CONDITIONS
+SDS CIRCUIT

ARTIFICIAL FLUID MODEL
(DENSITY, CONSISTENCY)

INLET/OUTLET

JAPANESE LAID-OPEN
PUBLICATION NO.2019-76327
MEDICINE INJECTION CONTAINER

JAPANESE LAID-OPEN PUBLICATION
NO.2021-72926
URINARY BLADDER URINE DYNAMIC
STATE MEASUREMENT DEVICE

Fig. 11

QUALITY CONTROL SYSTEM FOR SUSTAINED DRAIN SYSTEM CIRCUIT

SUSTAINED DRAIN SYSTEM CIRCUIT
• CLINICAL LOOP MODEL CONDITIONS
• SUSTAINED DRAIN PRINCIPLES
  (SIPHONING CONDITIONS)
• INITIAL SIPHONING PRESSURE & MINIMUM
  VOLUME DEFINITION
• CIRCUIT SPECIFICATION DEFINITION
  (LENGTH 1.2 m, φ 6 mm)

QUALITY CONTROL SYSTEM FOR SUSTAINED DRAIN SYSTEM CIRCUIT
• INITIAL SIPHONING PRESSURE CHECK METHOD
• SIMPLE COMPLETE DRAINING CHECK TEST METHOD
• MARGINAL TEST WITH VARIOUS FLUID PROPERTIES
• REFERENCE ARTIFICIAL URINARY BLADDER
  (VOLUME 50 ml, INTERNAL PRESSURE 25 cm WATER COLUMN)
• REFERENCE CONCENTRATED URINE SUGAR (NaCl 0.35 g/dl, Glc 5.4 g/dl)
• SCIENTIFIC QUALITY ASSURANCE AND QUALITY CONTROL
• CURRENT STATUS CHECK, OBJECT, TARGET, PDCA CYCLE

CURRENT STATUS CHECK: GRASPING OF ACTUAL CLINICAL USE
                      CONDITIONS AND VARIABLE FACTORS
OBJECT: IMPROVEMENT IN QUALITY OF LIFE BY PROVISION OF
        APPROPRIATELY-FUNCTIONING PRODUCT
TARGET: DEVELOPMENT AND MANAGEMENT OF PRODUCT
        CONFORMING TO USE CONDITIONS OF CLINICAL SITE

**P** PRODUCT SPECIFICATION DEFINITION, QUALITY ASSURANCE, AND
    SPECIFICATION/QUALITY CONTROL ←PDCA CYCLE
↓   (CLINICAL LOOP MODEL CONDITIONS, MINIMUM VOLUME, INITIAL
**D** SIPHONING PRESSURE)

    DETERMINATION OF ELEMENTS FOR QUALITY ASSURANCE
↓   (METHOD AND MATERIAL)
    (INITIAL SIPHONING PRESSURE MEASUREMENT METHOD, SIMPLE COMPLETE
↓   DRAIN CHECK TEST METHOD, REFERENCE ARTIFICIAL URINARY BLADDER,
    STANDARD REFERENCE URINE)
**C** SPECIFICATION CHECK & IMPLEMENTATION OF LOADING TEST
    METHOD (PRODUCT, CONDITIONS, MATERIAL ELEMENTS)
↓   (REFERENCE CONCENTRATED URINE SUGAR, VARIOUS
    CONSISTENCIES, DENSITY, SUGAR, PROTEIN, BLOOD)
**A** REFLECT ON PRODUCT, CONDITIONS, TEST METHOD, MATERIAL,
    AND SYSTEM COMPONENTS

Fig. 12

Europäisches
Patentamt

European
Patent Office

Office européen
des brevets

# EUROPEAN SEARCH REPORT

Application Number

EP 22 21 0825

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 084 593 A (JARUND HARRY SIGURD VALDEMAR) 18 April 1978 (1978-04-18) * column 2, line 54 – column 3, line 14 * ----- | 1-10 | INV. A61M1/00 A61F5/44 A61M25/00 |
| X | WO 2021/183365 A2 (BARD INC C R [US]) 16 September 2021 (2021-09-16) * paragraphs [0043], [0022]; figures 1,2 * ----- | 1-10 | |
| X | GB 1 120 557 A (ANDERSEN PROD H W) 17 July 1968 (1968-07-17) * page 2, line 10 – line 39; figure 1 * ----- | 1-10 | |
| X | US 2 602 448 A (MCKENNA WILLIAM F) 8 July 1952 (1952-07-08) * claim 1; figures * ----- | 1-10 | |
| X | EP 0 041 487 A1 (SCHOLANDER HANS PETER [SE]; SCHOLANDER AXEL FREDRIK [SE]) 9 December 1981 (1981-12-09) * page 15, line 34 – page 16, line 11; figure 1 * ----- | 1-10 | TECHNICAL FIELDS SEARCHED (IPC) A61M A61F |

The present search report has been drawn up for all claims

| Place of search | Date of completion of the search | Examiner |
|---|---|---|
| The Hague | 12 April 2023 | Lakkis, Angeliki |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 21 0825

This annex lists the patent family members relating to the patent documents cited in the above-mentioned European search report.
The members are as contained in the European Patent Office EDP file on
The European Patent Office is in no way liable for these particulars which are merely given for the purpose of information.

12-04-2023

| Patent document cited in search report | | Publication date | Patent family member(s) | | | Publication date |
|---|---|---|---|---|---|---|
| US 4084593 | A | 18-04-1978 | DE | 2616290 | A1 | 28-10-1976 |
| | | | GB | 1502734 | A | 01-03-1978 |
| | | | JP | S51133997 | A | 20-11-1976 |
| | | | US | 4084593 | A | 18-04-1978 |
| WO 2021183365 | A2 | 16-09-2021 | NONE | | | |
| GB 1120557 | A | 17-07-1968 | GB | 1120557 | A | 17-07-1968 |
| | | | US | 3503401 | A | 31-03-1970 |
| US 2602448 | A | 08-07-1952 | NONE | | | |
| EP 0041487 | A1 | 09-12-1981 | EP | 0041487 | A1 | 09-12-1981 |
| | | | ES | 8203617 | A1 | 01-04-1982 |
| | | | WO | 8103427 | A1 | 10-12-1981 |

EPO FORM P0459

For more details about this annex : see Official Journal of the European Patent Office, No. 12/82

**REFERENCES CITED IN THE DESCRIPTION**

**Patent documents cited in the description**

- JP 2019076327 A

- JP 2021072926 A